# EUROPEAN PATENT SPECIFICATION

(11) **EP 4 578 399 B1**
(45) Date of publication and mention of the grant of the patent: **15.07.2026**
(21) Application number: 24223330.2
(22) Date of filing: 27.12.2024
(51) Int. Cl.: A61B 6/03, A61B 6/04, A61B 6/00

(54) **MEDICAL SYSTEMS AND METHODS**
MEDIZINISCHE SYSTEME UND VERFAHREN
SYSTÈMES ET PROCÉDÉS MÉDICAUX

(30) Priority: 28.12.2023 CN 202311848033
(43) Date of publication of application: 02.07.2025
(73) Proprietor: Shanghai United Imaging Healthcare Co., Ltd., Shanghai 201807 (CN)
(72) Inventor: DU, Xinyue, Shanghai, 201807 (CN); WANG, Guanqun, Shanghai, 201807 (CN); HU, Qiwen, Shanghai, 201807 (CN); XIANG, Jun, Shanghai, 201807 (CN)
(74) Representative: Wang, Bo

(56) References cited:
- EP-A2- 3 699 622
- US-A1- 2021 236 076

## Description

### TECHNICAL FIELD

The present disclosure relates to the technical field of medical technology and, in particular, to a medical system and method.

### BACKGROUND

Imaging device, such as digital subtraction angiography (DSA) device, is essentially fixed mounted due to its large size and heavy weight, requiring patient access to a dedicated examination room or hybrid operating room. In hybrid operations, especially in some hybrid operations that are surgical, the imaging device is only required at certain stages of the operations, resulting in lower utilization of imaging device and increasing equipment cost for the hospital.

Therefore, there is a need to provide a medical system and method in order to increase the utilization of imaging device and reduce the equipment cost. Relevant background art is disclosed in the patent publications EP 3 699 622 A2 and US 2021/236076 A1.

### SUMMARY

The invention is defined by the appended claims.

One or more embodiments of the present disclosure provide a medical system, comprising: an equipment room and a plurality of medical rooms; an imaging device, the imaging device including a movable imaging component, the movable imaging component being shared by the plurality of medical rooms, and the movable imaging component being configured to move between the equipment room and the plurality of medical rooms; and a plurality of shielding doors, for each of the plurality of shielding doors, the shielding door being provided between one of the plurality of medical rooms and the equipment room.

One or more embodiments of the present disclosure provide a method for controlling a medical system, the medical system comprising: an equipment room and a plurality of medical rooms; an imaging device, the imaging device including a movable imaging component, the movable imaging component being shared by the plurality of medical rooms, and the movable imaging component being configured to move between the equipment room and the plurality of medical rooms; a plurality of shielding doors, for each of the plurality of shielding doors, the shielding door being provided between one of the plurality of medical rooms and the equipment room; and a controller; the method comprising: in response to determining that the movable imaging component needs to move to a target medical room of the plurality of medical rooms, controlling, by the controller, the movable imaging component to move from the equipment room to the target medical room through a target shielding door of the plurality of shielding doors corresponding to the target medical room; controlling, by the controller, the movable imaging component to perform a scan process on an object in the target medical room; and in response to determining that the movable imaging component needs to move to the equipment room, controlling, by the controller, the movable imaging component to move from the target medical room to the equipment room through the target shielding door.

One or more embodiments of the present disclosure provide a non-transitory computer readable medium, comprising executable instructions, wherein when executed by one or more processors of a computing device, the executable instructions cause the computing device to perform a method for controlling a medical system, the medical system comprising: an equipment room and a plurality of medical rooms; an imaging device, the imaging device including a movable imaging component, the movable imaging component being shared by the plurality of medical rooms, and the movable imaging component being configured to move between the equipment room and the plurality of medical rooms; a plurality of shielding doors, for each of the plurality of shielding doors, the shielding door being provided between one of the plurality of medical rooms and the equipment room; and a controller; the method including: in response to determining that the movable imaging component needs to move to a target medical room of the plurality of medical rooms, controlling, by the controller, the movable imaging component to move from the equipment room to the target medical room through a target shielding door of the plurality of shielding doors corresponding to the target medical room; controlling, by the controller, the movable imaging component to perform a scan process on an object in the target medical room; and in response to determining that the movable imaging component needs to move to the equipment room, controlling, by the controller, the movable imaging component to move from the target medical room to the equipment room through the target shielding door.

### BRIEF DESCRIPTION OF THE DRAWINGS

FIG. 1 is a top view of an exemplary medical system according to some embodiments of the present disclosure;
FIG. 2 is a schematic diagram of an exemplary imaging device according to some embodiments of the present disclosure;
FIG. 3 is a top view of an exemplary medical system according to some embodiments of the present disclosure;
FIG. 4 is a top view of an exemplary medical system according to some embodiments of the present disclosure;
FIG. 5A is a top view of an exemplary medical system according to some embodiments of the present disclosure;
FIG. 5B is a top view of an exemplary medical system according to some embodiments of the present disclosure;
FIG. 6 is a flowchart of an exemplary process for controlling a medical system according to some embodiments of the present disclosure; and
FIG. 7 is a flowchart of an exemplary process for controlling a movable imaging component to move to a target medical room according to some embodiments of the present disclosure.

### DETAILED DESCRIPTION

Traditionally, an imaging device is essentially fixed mounted, one imaging device can only be used in one medical room, the utilization rate of imaging device is low, and the equipment cost for the hospital is high. In some embodiments, the imaging device includes a DSA device, a positron emission tomography (PET) device, a computed tomography (CT) device, a magnetic resonance imaging (MRI) device, or the like. For the sake of illustration, the following descriptions are all illustrated with DSA device as an example.

DSA device aids in applications in the fields of angiography, cardiology, and neurology. Existing DSA devices, whether in conventional catheterization rooms or hybrid operating rooms, may only be fixed to a specific area of the room, and the DSA devices may only be utilized around a designated medical bed. In hybrid operating rooms, where the DSA device is used only at a certain period of time during or after the operations, and the DSA device is parked in the operating rooms most of the time. In this case, the type of layout described above results in very low utilization of DSA device, which greatly increases equipment cost. In order to share the same imaging device across a plurality of operating rooms and thus reduce cost for the hospital, the DSA device needs to support a sufficiently large range of motion, such as the ability to move from one operating room to another operating room. Because of the large size and weight of the C-arm of the DSA device, it is difficult to design the DSA device to be easily transported as a movable imaging device to move from one operating room to another operating room, and/or reducing the size of the C-arm may greatly limit the clinical use of the DSA device.

A medical system shown in some embodiments of the present disclosure includes an equipment room, a plurality of medical rooms, an imaging device, and a plurality of shielding doors. The medical system is capable of allowing the plurality of medical rooms to share the imaging device.

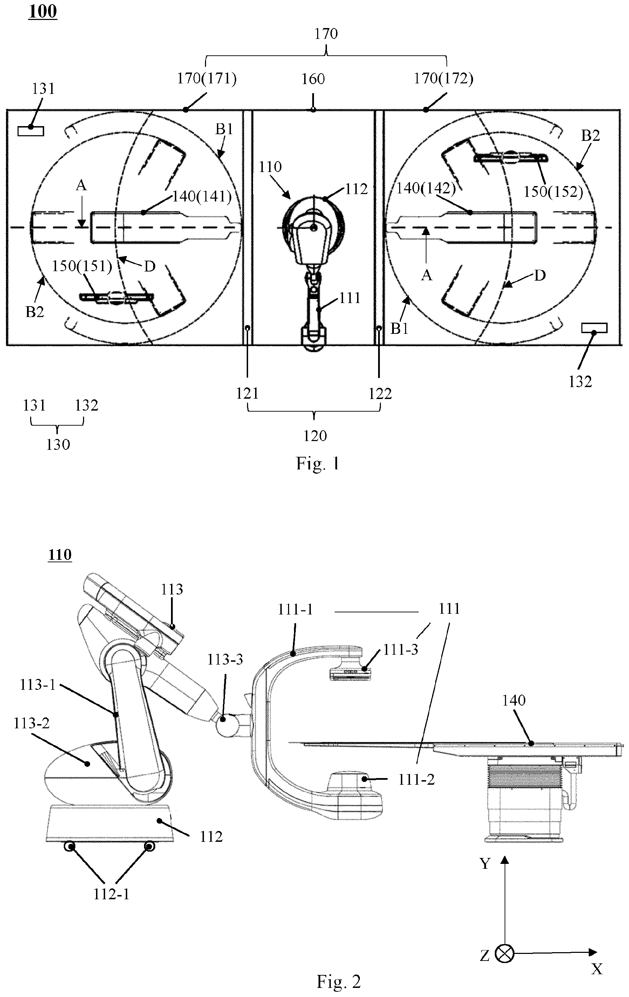

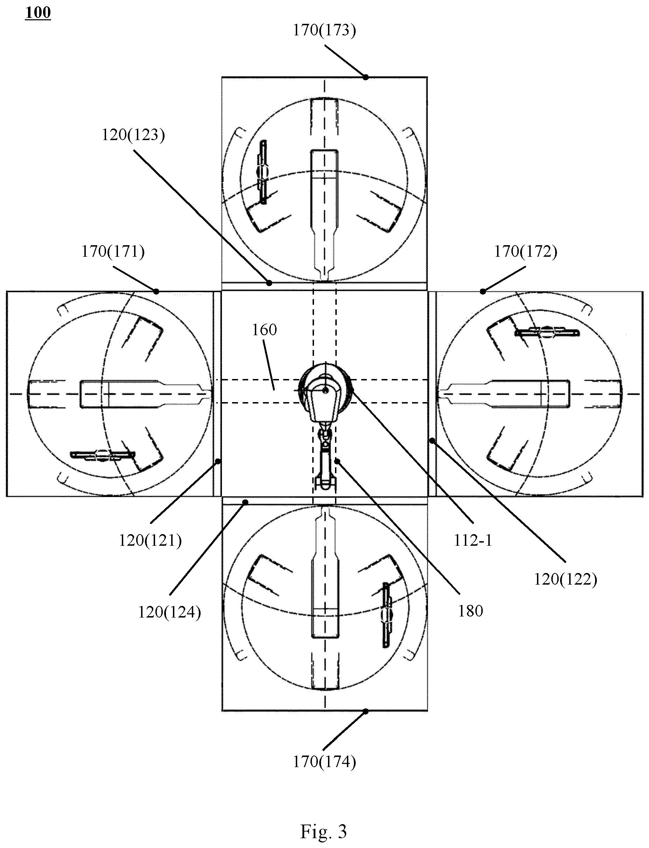

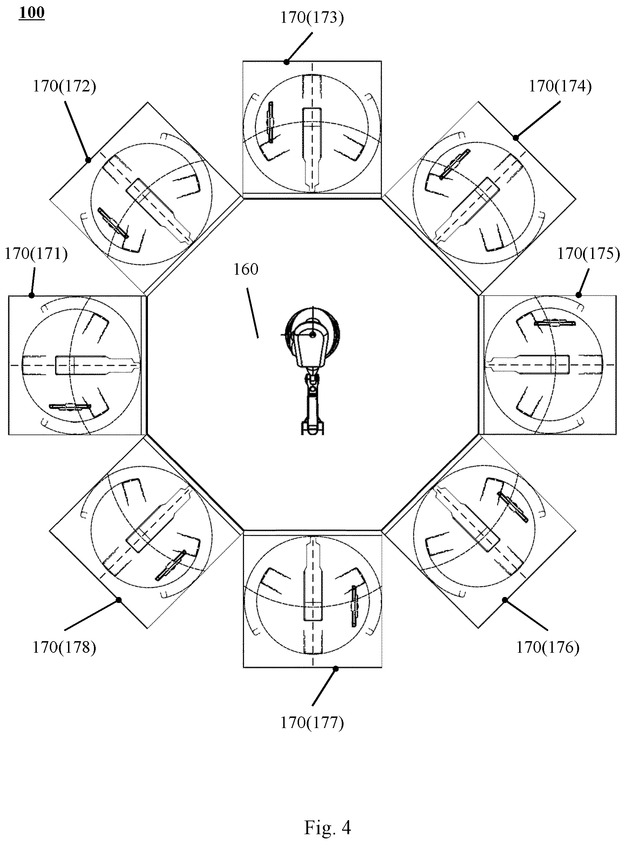

FIG. 1 is a top view of an exemplary medical system according to some embodiments of the present disclosure; FIG. 2 is a schematic diagram of an exemplary imaging device according to some embodiments of the present disclosure; FIG. 3 is a top view of an exemplary medical system according to some embodiments of the present disclosure; FIG. 4 is a top view of an exemplary medical system according to some embodiments of the present disclosure. As shown in FIG. 2, the Y direction is vertical direction, and the X and Z directions are horizontal and perpendicular to the Y direction. The X direction is a direction in which a movable imaging component 111 moves to be away from or close to a base 112, and the Z direction is perpendicular to the X direction. The X, Y, and Z directions form an orthogonal coordinate system related to an imaging device 110.

In some embodiments, a medical system includes an equipment room, a plurality of medical rooms, the imaging device, and a plurality of shielding doors. The imaging device includes a movable imaging component. The movable imaging component is shared by the plurality of medical rooms, and the movable imaging component is configured to move between the equipment room and the plurality of medical rooms. For each of the plurality of shielding doors, the shielding door is provided between one of the plurality of medical rooms and the equipment room. For example, as shown in FIG. 1, a medical system 100 includes an equipment room 160, two medical rooms 170, the imaging device 110, and two shielding doors 120. The imaging device 110 includes the movable imaging component 111. The movable imaging component 111 is shared by the two medical rooms 170, and the movable imaging component 111 is configured to move between the equipment room 160 and the two medical rooms 170. For each of the two shielding doors 120, the shielding door 120 is provided between one of the two medical rooms 170 and the equipment room 160.

The equipment room 160 is a place for accommodating the imaging device 110. In some embodiments, the count (number) of equipment rooms 160 is at least one. For example, the count (number) of equipment rooms 160 is 1, 2, 3, or the like. In some embodiments, the shape of the equipment room 160 may include rectangular prisms, triangular prisms, quadrilateral prisms, hexagonal prisms, octagonal prisms, etc., which is not limited in the present disclosure.

It should be noted that when the medical system 100 includes a plurality of equipment rooms 160, the movable imaging component 111 moves between the plurality of equipment rooms 160 and the plurality of medical rooms 170 in a similar manner as the condition when the number of equipment rooms 160 is a single one. For ease of description, the following descriptions are all illustrated by taking one equipment room 160 as an example.

The medical room 170 is a place where medical activities (e.g., diagnosis, treatment, examination, imaging, or the like) are performed. The medical room 170 includes an operating room or an examination room.

The operating room is a place where operations are performed. For example, the operating room includes a hybrid operating room. The hybrid operating room realizes the combination of minimally invasive interventional operation and traditional surgical open operation through the full integration of the imaging device 110 with surgery in a class 100 laminar-flow operating room to solve a plurality of types of complex operations and reduce operation risk, and save operation time. The hybrid operation has a wide range of applications in neurological, cardiac, and/or vascular fields.

The examination room is a place where patients are examined. In the examination room, medical images of patients are determined by scanning the patients through the imaging device 110.

In some embodiments, the number of medical rooms 170 is at least two. For example, the number of medical rooms 170 is 2, 3, 5, 8, or the like. In some embodiments, the shape of the medical rooms 170 includes rectangular prisms, triangular prisms, quadrilateral prisms, hexagonal prisms, octagonal prisms, etc., which is not limited in the present disclosure.

In some embodiments, the plurality of medical rooms 170 are arranged around the equipment room 160. For example, as shown in FIG. 1, a first medical room 171 and a second medical room 172 are arranged around the equipment room 160. For another example, as shown in FIG. 3, the first medical room 171, the second medical room 172, a third medical room 173, and a fourth medical room 174 are arranged around the equipment room 160. For another example, as shown in FIG. 4, the first medical room 171, the second medical room 172, the third medical room 173, the fourth medical room 174, a fifth medical room 175, a sixth medical room 176, a seventh medical room 177, and an eighth medical room 178 are arranged around the equipment room 160.

The plurality of medical rooms 170 are arranged around the equipment room 160, indicating that the layout of the plurality of medical rooms 170 is centered around the equipment room 160. The equipment room 160 serves as a central area configured to accommodate the imaging device 110. The movable imaging component 111 may be movable between the equipment room 160 and the plurality of medical rooms 170.

In some embodiments, the number and the arrangement of the plurality of medical rooms 170 may be varied.

In some embodiments, as shown in FIG. 1, the medical system 100 includes two medical rooms 170, the first medical room 171 and the second medical room 172, and the first medical room 171 and the second medical room 172 are located on two sides of the equipment room 160, respectively.

In some embodiments, as shown in FIG. 3, the number of medical rooms 170 is four and the medical rooms 170 are located on the four sides of the equipment room 160, respectively. That is, the four medical rooms 170 are the first medical room 171, the second medical room 172, the third medical room 173 and the fourth medical room 174. The first medical room 171 and the second medical room 172 are located on two opposite sides of the equipment room 160, respectively, and the third medical room 173 and the fourth medical room 174 are located on the other two opposite sides of the equipment room 160, respectively.

In some embodiments, as shown in FIG. 4, the number of medical rooms 170 is eight and the medical rooms 170 are located on the eight sides of the equipment room 160, respectively. That is, the eight medical rooms 170 are the first medical room 171, the second medical room 172, the third medical room 173, the fourth medical room 174, the fifth medical room 175, the sixth medical room 176, the seventh medical room 177, and the eighth medical room 178. The equipment room 160 with a shape of octagonal prism includes four sets of sides each of which includes two opposite sides. The first medical room 171 and the fifth medical room 175 are located on a first set of two opposite sides of the equipment room 160, respectively, the second medical room 172 and the sixth medical room 176 are located on a second set of two opposite sides of the equipment room 160, respectively, the third medical room 173 and the seventh medical room 177 are located on a third set of two opposite sides of the equipment room 160, respectively, and the fourth medical room 174 and the eighth medical room 178 are located on a fourth set of two opposite sides of the equipment room 160, respectively.

In the present disclosure, through different arrangements between the equipment room 160 and the plurality of medical rooms 170, the single imaging device 110 can be shared by different numbers of medical rooms.

The imaging device 110 refers to a device used to acquire medical images of a region of interest (ROI) of a scanned subject (also referred to as scanned object) (e.g., a patient, an examinee, or the like). More on the imaging device 110 can be found above and in its related description.

In some embodiments, the imaging device 110 is a floor-mounted DSA device. The floor-mounted DSA device is a DSA device in which a base 112 for supporting the movable imaging component 111 is placed on the floor. In some embodiments, the imaging device 110 is a suspended DSA device. The suspended DSA device is a DSA device in which the base for supporting the movable imaging component 111 is placed on the ceiling. A detailed description of the base 112 is provided below.

In some embodiments, the imaging device 110 includes the movable imaging component 111.

In the case where the imaging device 110 is a DSA device, the movable imaging component 111 refers to a component that is movable and configured to acquire a medical image of a region of interest of a scanned object.

In some embodiments, as shown in FIG. 2, the movable imaging component 111 includes a C-arm 111-1, a radiation source 111-2, and a detector 111-3.

The radiation source 111-2 is mounted at one end of the C-arm 111-1, and the detector 111-3 is mounted at the other end of the C-arm 111-1, so as to make the radiation source 111-2 and the detector 111-3 opposite to each other.

The radiation source 111-2 is a device capable of emitting rays such as X-rays, gamma rays, or electron rays.

The detector 111-3 is a device capable of receiving rays emitted by the radiation source 111-2. Operations such as medical examinations or treatments can be realized with the cooperation of the radiation source 111-2 and the detector 111-3.

In some embodiments, the radiation source 111-2 includes a bulb tube, which is capable of emitting rays (e.g., X-rays). The detector 111-3 may be a flat panel detector, and the rays emitted by the bulb tube pass through the patient and are received by the detector 111-3. The received X-ray signals are converted into electrical signals to create imaging data. An image processing system obtains a clear image of a blood vessel through subtractions, enhancements, and reimaging processes based on the imaging data. The image processing system may be a controller 130, or other system having image processing capabilities.

In some embodiments, as shown in FIG. 2, the imaging device 110 may include a base 112. The movable imaging component 111 is connected to the base 112, and the movable imaging component 111 is movable relative to the base 112.

The base 112 refers to a component for supporting the movable imaging component 111. In some embodiments, the shape of the base 112 may include a rectangular, cylindrical, prismatic, or the like, and is not limited by the present disclosure.

The movable imaging component 111 and the base 112 may be connected in a variety of ways. In some embodiments, the movable imaging component 111 is movably connected to the base 112, such that the movable imaging component 111 can move relative to the base 112.

In some embodiments, the movement of the movable imaging component 111 relative to the base 112 includes rotation, linear and/or curvilinear movement, or the like, ensuring that the movable imaging component 111 can move over a spatial range relative to the base 112. For example, the movable imaging component 111 is connected to the base 112 via a rotating vice, a rolling bearing, a cardan joint, or the like, thereby realizing the movement of the imaging component 111 relative to the base 112. Rotations of the movable imaging component 111 relative to the base 112 include at least one of rotation of the movable imaging component 111 relative to the base 112 around the vertical direction (e.g., in the Y direction as shown in FIG. 2), rotation of the movable imaging component 111 relative to the base 112 along a circumferential direction of the C-arm (e.g., around the Z direction as shown in FIG. 2), and/or rotation of the movable imaging component 111 relative to the base 112 around the X direction as shown in FIG. 2.

According to the claimed subject matter, the movable imaging component 111 is connected to the base 112 by means of a robotic arm (e.g., a robotic arm 113-1 as shown in FIG. 2) having a plurality of degrees of freedom. In some embodiments, the robotic arm 113-1 includes a four-degree-of-freedom robotic arm, a six-degree-of-freedom robotic arm, a seven-degree-of-freedom robotic arm, or the like.

In some embodiments, the plurality of degrees of freedom of the robotic arm 113-1 is realized based on the mechanical structure of the robotic arm 113-1 itself, and the robotic arm 113-1 comprises a plurality of mechanical joints sequentially connected, with two adjacent mechanical joints connected to each other by a joint axis.

In some embodiments, the C-arm 111-1 is connected to the robotic arm 113-1, ensuring that the movable imaging component 111 can have a higher degree of freedom of flexibility in the spatial range when driven by the robotic arm 113-1. This facilitates the movement of the movable imaging component 111 between different medical rooms 170 under the driving of the robotic arm 113-1, as well as realizing the relative movement between the movable imaging component 111 and a medical bed 140. The connection method between the C-arm 111-1 and the robotic arm 113-1 include, but are not limited to, fixing by bolting, fixing by welding, or the like, which is not limited in the present disclosure. More about the medical bed 140 can be found in FIG. 4 and its related description.

In some embodiments, the robotic arm 113-1 is connected to the base 112, the movable imaging component 111 through the robotic arm 113-1 to move (e.g., rotate, move in a straight line or a curve over a spatial range, or the like) relative to the base 112. The connection method between the robotic arm 113-1 and the base 112 include, but are not limited to, fixing by bolting, fixing by welding, or the like, which is not limited in the present disclosure. It should be noted that during movement of the movable imaging component 111 between the equipment room 160 and the medical rooms 170, the state of the C-arm 111-1 of the movable imaging component 111 is a vertical state, i.e., a line connecting the radiation source 111-2 and the detector 111-3 is perpendicular to the horizontal plane. It will be appreciated that during movement of the movable imaging component 111 between the equipment room 160 and the medical rooms 170, the state of the C-arm 111-1 of the movable imaging component 111 may also be other states, for example, the line connecting the radiation source 111-2 and the detector 111-3 on the C-arm 111-1 may also not be perpendicular to the horizontal plane. As shown in FIG. 1, with the base 112 located in the equipment room 160, after the movable imaging component 111 enters a medical room 170, the rotation of the robotic arm 113-1 in a maximally extended state around the vertical direction in the medical room 170 is shown by a rotational movement trajectory D. The maximally extended state means that all of the joints of the robotic arm 113-1 are extended to the furthest position the robotic arm 113-1 can reach, which is also referred to as the maximum working range of the robotic arm 113-1.

It will be appreciated that the robotic arm 113-1 can extend and retract, thereby increasing or decreasing the size of the C-arm 111-1 in the spatial range. In some embodiments, when the movable imaging component 111 is not in work and located in the equipment room 160, the robotic arm 113-1 is in a folded state in the equipment room 160, allowing for the DSA device to be placed in the equipment room 160 with a smaller volume. During a process for transporting the imaging device 110, the robotic arm 113-1 may also be in the folded state.

The folded state is a state in which the robotic arm 113-1 folds or retracts various joints and limbs in a certain way to occupy a smaller volume or facilitate storage.

In some embodiments of the present disclosure, by connecting the movable imaging component 111 and the base 112 via the robotic arm 113-1, the movement of the movable imaging component 111 relative to the of the base 112 is more flexible, and the movable imaging component 111 has a greater movement range.

In some embodiments, the robotic arm 113-1 belongs to a robot 113. the robot 113 includes a robot base 113-2, the robotic arm 113-1, and a robotic arm end tool 113-3. The robot base 113-2 provides stability and support for the robot 113, the robotic arm 113-1 executes tasks with its plurality of mechanical joints sequentially connected, and the robotic arm end tool 113-3, connected to the robotic arm's end, performs the specific task (such as gripping, welding, or cutting). The connection between the robot base 113-2, the robotic arm 113-1, and the robotic arm end tool 113-3 includes mechanical linkages.

In some embodiments, the C-arm 111-1 is connected to the robotic arm end tool 113-3 of the robot 113, the robot base 113-2 of the robot 113 is provided on the base 112, and the robot 113 has the robotic arm 113-1 having a plurality of degrees of freedom, including degrees of freedom of movement and degrees of freedom of rotation.

In some embodiments, the base 112 may be fixed within the equipment room 160. For example, the base 112 is fixed to the floor within the equipment room 160. For another example, the base 112 is fixed to the ceiling within the equipment room 160.

Fixing methods for the base 112 include, but are not limited to, fixing by bolting, fixing by welding, or the like, which is not limited in the present disclosure.

In some embodiments, when the base 112 is fixed within the equipment room 160, the movable imaging component 111 may be moved between the equipment room 160 and the plurality of medical rooms 170 by means of the C-arm 111-1 and/or the robotic arm 113-1.

As an example, the robotic arm 113-1 may realize movement of the movable imaging component 111 between the equipment room 160 and the medical room 170 through actions such as extending, retracting, lifting and falling, rotating around the vertical direction, or the like. When the movable imaging component 111 needs to enter the medical room 170 from the equipment room 160, the robotic arm 113-1 may be gradually extended so that the movable imaging component 111 passes through an opened shielding door 120 corresponding to the medical room 170 and enters the medical room 170. When the movable imaging component 111 needs to return from the medical room 170 to the equipment room 160, the robotic arm 113-1 may be gradually retracted so that the movable imaging component 111 returns to the equipment room 160 through the opened shielding door 120, and then the robotic arm 113-1 stays in the folded state in the equipment room 160.

Based on the layout between the equipment room 160 and the plurality of medical rooms 170, the DSA device is mounted in the equipment room 160 through the base 112, and the controller 130 may control the movable imaging component 111 to move relative to the base 112, thereby entering the corresponding medical room 170 through the opened shielding door 120.

In some embodiments, the base 112 may not be fixed within the equipment room 160. As shown in FIG. 2, the base 112 of the imaging device 110 further includes a movable device 112-1. The movable device 112-1 includes at least one of wheels and a track connecting device. The movable device 112-1 is configured to enable the base 112 to move. The track connecting device is a device for connecting and/or docking to a track.

In some embodiments, when the base 112 includes the movable device 112-1, the base 112 is not fixed within the equipment room 160, and the base 112 can move between the equipment room 160 and the plurality of medical rooms 170.

In some embodiments, when the movable device 112-1 is wheels, the base 112 can move anywhere in the equipment room 160 and the medical rooms 170.

In some embodiments, as shown in FIG. 3, a track 180 is also disposed between the equipment room 160 and the plurality of medical rooms 170. The track 180 is configured to connect the track connecting device and provide a path for movement of the track connecting device.

The track 180 is used to limit a movement path of the track connecting device. In some embodiments, the track 180 may be a straight track (as shown in FIG. 3), a curved track, a looped track, or other shaped track. In some embodiments, the track 180 is disposed on the floor of the equipment room 160 and the plurality of medical rooms 170. In some embodiments, the track 180 is disposed on the ceiling of the equipment room 160 and the plurality of medical rooms 170.

In some embodiments, the track connecting device is provided with rollers, which roll along the track 180, thereby driving the imaging device 110 along the track 180. In other embodiments, the track connecting device is provided with a slider, and the track 180 includes a slide groove for the slider to slide, and the slider slides along the track 180, thereby driving the imaging device 110 to move along the track 180.

It should be noted that when the base 112 of the imaging device 110 further includes the movable device 112-1, the movable imaging component 111 may not be connected to the base 112 via the robotic arm 113-1 with the plurality of degrees of freedom. In other words, the robotic arm 113-1 with the plurality of degrees of freedom does not need to be provided. Instead, the movable imaging component 111 can be moved between the equipment room 160 and the plurality of medical rooms 170 directly through the movable device 112-1. In some embodiments, during movement of the movable imaging component 111 between the equipment room 160 and the plurality of medical rooms 170, the base 112 is also moved between the equipment room 160 and the plurality of medical rooms 170. By the movement of the movable imaging component 111 by the movable device 112-1, the movable imaging component 111 may move between the equipment room 160 and the plurality of medical rooms 170.

In some embodiments, during movement of the movable imaging component 111 between the equipment room 160 and the plurality of medical rooms 170, the base 112 is also moved between the equipment room 160 and the plurality of medical rooms 170. By combining the movement of the movable imaging component 111 relative to the base 112 by the robotic arm 113-1 and the movement of the movable imaging component 111 by the movable device 112-1, the movable imaging component 111 may move between the equipment room 160 and the plurality of medical rooms 170.For example, the base 112 first moves by the movable device 112-1, and after the base 112 is moved to a designated position in the medical room 170, the movable imaging component 111 is then controlled in conjunction with the robotic arm 113-1 to move relative to the base 112 so that the movable imaging component 111 is located at a certain position to image the scanned object.

According to the claimed subject matter, during movement of the movable imaging component 111 between the equipment room 160 and the plurality of medical rooms 170, the base 112 is located in the equipment room 160, but not fixed within the equipment room 160. For example, a plurality of predetermined locations in the equipment room 160 may be predetermined, and the base 112 may be moved between the plurality of predetermined locations by means of wheels, tracks, or the like, which may allow the movable imaging component 111 to have a greater movable range. As an example, the predetermined locations in the equipment room 160 may include the center of the equipment room 160 and the doorway of each of the shielding doors 120, and the base 112 may be moved between the center of the equipment room 160 and the doorway of each of the shielding doors 120. As another example, the base 112 may be freely movable in the equipment room 160 by means of wheels or the like, which may further enable the movable imaging component 112 to have a greater movement range. In this case, the track 180 may be disposed in the equipment room 160 and the plurality of medical rooms 170, or only in the equipment room 160. As an example, after the base 112 moves to a designated position (e.g., the doorway of a target medical room) in the equipment room 160 by the movable device 112-1, the movable imaging component 111 is moved into the target medical room by controlling the movement of the movable imaging component 111 relative to the base 112 in conjunction with the robotic arm 113-1 (e.g., by extension of the robotic arm 113-1).

In some embodiments, the base 112 with the movable device has the ability of moving between the equipment room 160 and the plurality of medical rooms 170 or in the equipment room 160. However, in some cases (e.g., the space in the target medical room is limited), during movement of the movable imaging component 111 between the equipment room 160 and the plurality of medical rooms 170, the base 112 may be set to be fixed within the equipment room 160 (e.g., at a designated position, such as the center of the equipment device 160). For example, a locking assembly may be utilized to lock the movable device to make the movable device (the base 112) unable to move.

In some embodiments of the present disclosure, the base of the imaging device is movable, which expands the movement range of the imaging device, thereby further supporting the sharing of a single imaging device by a plurality of medical rooms.

In some embodiments, the equipment room 160 includes a lifting platform 161, and the lifting platform 161 is configured to carry the imaging device 100 to different floors.

The lifting platform 161 refers to a device that can adjust the floor on which the imaging device 110 is located. In some embodiments, the lifting platform 161 may include a device such as a vertical conveyor, an elevator, or the like.

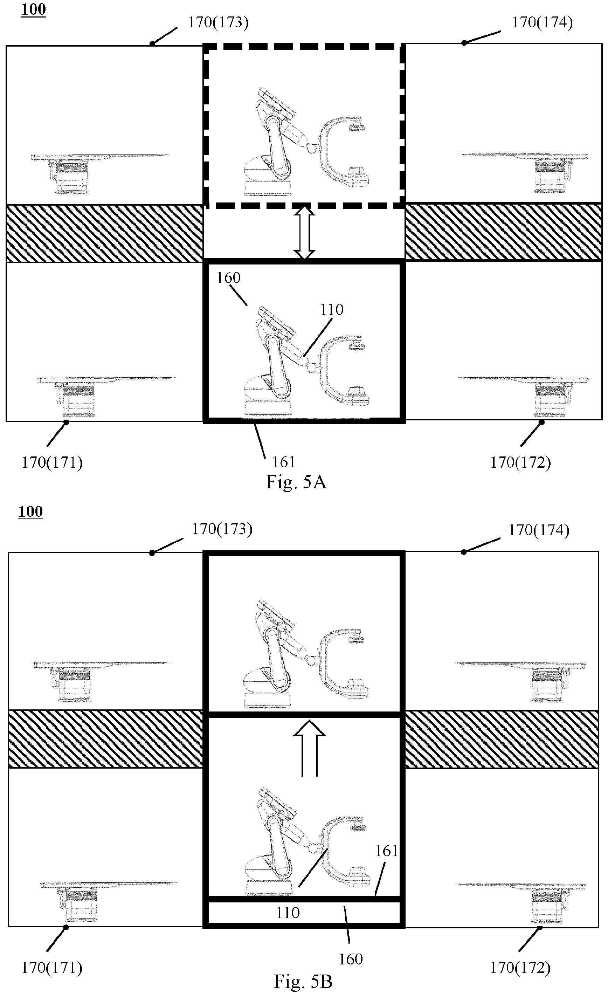

In some embodiments, the plurality of medical rooms 170 are distributed across a plurality of floors. The lifting platform 161 can drive the equipment room 160 up and down to adjust the floor where the imaging device 110 is located. As shown in FIG. 5A, the first medical room 171 and the second medical room 172 are located on the first floor, and the third medical room 173 and the fourth medical room 174 are located on the second floor. The lifting platform 161 drives the equipment room 160 to move between the two floors.

In some embodiments, the plurality of medical rooms 170 are distributed across a plurality of floors. The lifting platform 161 may drive the imaging device 110 up and down in the equipment room 160 to adjust the floor where the imaging device 110 is located, but the equipment room 160 does not move. As shown in FIG. 5B, the first medical room 171 and the second medical room 172 are located on the first floor, and the third medical room 173 and the fourth medical room 174 are located on the second floor. The height of the equipment room 160 may be the same as the sum of the heights of the two floors, and the lifting platform 161 drives the imaging device 110 up and down in the equipment room 160 so as to realize that the imaging device 110 is moved between the two floors.

In some embodiments of the present disclosure, adjusting the floor where the imaging device is located by means of a lifting platform can realize the sharing of a single imaging device by a plurality of medical rooms on a plurality of floors to further increase the utilization rate of the imaging device.

In some embodiments, when the base of the imaging device remains stationary within the equipment room during movement of the movable imaging component between the equipment room and the medical room, the movement trajectory of the imaging device includes a movement trajectory of the movable imaging component, e.g., a movement speed, movement direction, movement time, movement distance, or the like of the imaging device. In some embodiments, the movement of the movable imaging component includes rotation around the vertical direction and extending and retracting movement of the robotic arm, the movement speed includes the movement speed and the rotation speed of the end of the robotic arm, and the movement direction includes the movement direction and the rotation direction of the end of the robotic arm.

In some embodiments, when the base moves between the equipment room and the medical room or within the equipment room as the movable imaging component moves between the equipment room and the medical room, the movement trajectory of the imaging device includes a movement trajectory of the movable imaging component and a movement trajectory of the movable device (the movement trajectory of the base). The movement trajectory of the movable imaging component includes the movement speed (i.e., the movement speed and the rotation speed of the end of the robotic arm), the movement direction (i.e., the motion direction and the rotation direction of the end of the robotic arm), the movement time, or the like, of the movable imaging component, and the movement trajectory of the movable device includes the movement speed, the movement direction, the movement time, the movement distance, or the like, of the movable device. Since the movement of the movable device is in the form of movement (e.g., linear and/or curvilinear movement) on the horizontal plane (floor or ceiling) and rotation about the vertical direction, the movement speed of the movable device includes the movement speed and the rotation speed on the horizontal plane, and the movement direction of the movable device includes the movement direction and the rotation direction on the horizontal plane.

The shielding door 120 refers to a door that is used to realize the separation of the equipment room 160 and the corresponding medical room 170. In some embodiments, the shielding door 120 is configured to shield radiation. For example, the shielding door 120 is made of lead, tungsten alloy, copper alloy, or the like, .

In some embodiments, the plurality of shielding doors 120 corresponds to the plurality of medical rooms 170. For example, each of the plurality of shielding doors 120 corresponds to one of the plurality of medical rooms 170, and a shielding door 120 is provided between the equipment room 160 and the corresponding medical room 170. The equipment room 160 may also be understood to be enclosed by the plurality of shielding doors 120 and/or walls. For example, as shown in FIG. 1, a first shielding door 121 corresponds to the first medical room 171, and a second shielding door 122 corresponds to the second medical room 172. The equipment room 160 is enclosed by the first shielding door 121, the second shielding door 122 and walls. As another example, as shown in FIG. 3, the first shielding door 121 corresponds to the first medical room 171, the second shielding door 122 corresponds to the second medical room 172, a third shielding door 123 corresponds to the third medical room 173, and a fourth shielding door 124 corresponds to the fourth medical room 174. The equipment room 160 is enclosed by the first shielding door 121, the second shielding door 122, the third shielding door 123, and the fourth shielding door 124.

In some embodiments, when the movable imaging component 111 needs to move to a target medical room of the plurality of medical rooms 170, a target shielding door of the plurality of shielding doors 120 between the target medical room and the equipment room 160 is configured to be open, and the rest of the plurality of shielding doors 120 are configured to be closed. Accordingly, the target medical room and the equipment room 160 form a sealed space, and the rest of the plurality of medical rooms 170 are isolated from the sealed space formed by target medical room and the equipment room 160.

The target medical room is the medical room 170 where the imaging device 110 is currently required to be used.

The target shielding door is the shielding door 120 corresponding to the target medical room. The shielding door 120 corresponding to the target medical room refers to the shielding door 120 between the target medical room and the equipment room 160. The rest of the plurality of shielding doors are shielding doors 120 corresponding to the medical rooms 170 where the imaging device 110 is not currently required to be used, i.e., the shielding doors corresponding to the medical rooms other than the target medical room. For example, as shown in FIG. 1, when the first medical room 171 is the target medical room, the first shielding door 121 is the target shielding door, and the second shielding door 122 is the rest of the plurality of shielding doors.

It will be appreciated that when the target shielding door is configured to be open and the rest of the plurality of shielding doors are configured to be closed, the target medical room and the equipment room 160 form a sealed space, i.e., the target medical room and the equipment room 160 are in communicate with each other to form an enclosed room. For example, as shown in FIG. 1, when the first shielding door 121 is the target shielding door and the second shielding door 122 is the rest of the plurality of shielding doors, the first shielding door 121 is open and the second shielding door 122 is closed, the equipment room 160 and the first medical room 171 corresponding to the first shielding door 121 are in communication with each other to form a sealed space, and the second medical room 172 is isolated from the sealed space formed by the first medical room 171 and the equipment room 160 by the closed second shielding door 122.

When the imaging device 110 is not in use, there is no limit to the state (e.g., open or closed) of each of the shielding doors 120, as long as it is ensured that when the imaging device 110 is used in the target medical room, the target shielding door is open and the rest of the plurality of shielding doors are closed. In some embodiments, the shielding doors 120 are closed by default when the imaging device 110 is not being used, and the target shielding door is open when the target medical room needs to use the imaging device 110. For example, when the imaging device 110 is not being used, the first shielding door 121 and the second shielding door 122 are both in the closed state. If the first medical room 171 needs to use the imaging device 110, the first shielding door 121 is open, and the second shielding door 122 remains the closed state. In some embodiments, the shielding doors 120 are in the open state by default when the imaging device 110 is not in use, and the rest of the plurality of shielding doors are closed when the target medical room needs to use the imaging device 110. For example, the first shielding door 121 and the second shielding door 122 are both in the open state. If the first medical room 171 needs to use the imaging device 110, the second shielding door 122 is closed, and the first shielding door 121 remains the open state.

In some embodiments, if the movable device of the base 112 is wheels, and when the imaging device 110 is being used in the target medical room, the base 112 is also located in the target medical room, the target shielding door may be in the closed state. The target medical room forms a sealed space and is isolated from the equipment room 160 and the rest of the plurality of medical rooms, and the rest of the plurality of the shielding doors may be in the closed or open state.

In some embodiments, the opening and closing of the shielding doors 120 is manually controlled by medical workers. In other embodiments, the opening and closing of the shielding doors 120 is controlled by the controller 130. More about the controller 130 can be found below and its related description.

In some embodiments of the present disclosure, providing the shielding doors 120 between the equipment room 160 and the medical rooms 170 ensures the independence of each medical room 170, and that the use of the imaging device 110 in the target medical room will not have an impact on the other medical rooms in the operations and/or examinations.

In some embodiments, as shown in FIG. 1, the medical system 100 further includes the controller 130, and the controller 130 is configured to control the plurality of shielding doors 120 to open or close, and/or to control movement of the movable imaging component 111 between the equipment room 160 and the plurality of medical rooms 170.

For example, the controller 130 may issue program instructions directly, or the controller 130 may process data and/or information obtained from other devices or components of the system to perform one or more of the functions described in the present disclosure. The controller 130 may also execute program instructions based on the data, information, and/or processing results.

In some embodiments, the controller 130 may be provided as a stand-alone hardware device in the medical room 170 and/or the equipment room 160, or the controller 130 may be integrally provided on other devices within the medical room 170. For example, the controller 130 is integrally provided on the imaging device 110, the medical bed 140, or a display 150.

In some embodiments, the controller 130 is communicated with the imaging device 110, and the controller 130 is communicated with drive structures of the shielding doors 120, thereby controlling the plurality of shielding doors 120 to open or close, or controlling the movement of the movable imaging component 111 between the equipment room 160 and the plurality of medical rooms 170. For example, the controller 130 issues instructions to the imaging device 110 and the drive structures of the shielding doors 120, respectively, to control the shielding doors 120 to open or close based on the issued instructions, or to control the movement of the movable imaging component 111 relative to the base 112 based on the issued instructions,, so that one of the plurality of shielding doors 120 can be controlled to open and the rest of the plurality of shielding doors 120 can be controlled to close, and the movable imaging component 111 can be controlled to enter the medical room 170 corresponding to the open shielding door 120 through the open shielding door 120. Once the movable imaging component 111 of the imaging device 110 enters the corresponding medical room 170, the movable imaging component 111 provides the doctor with image support of the patient for medical activities, such as minimally invasive interventional operations, or hybrid operations.

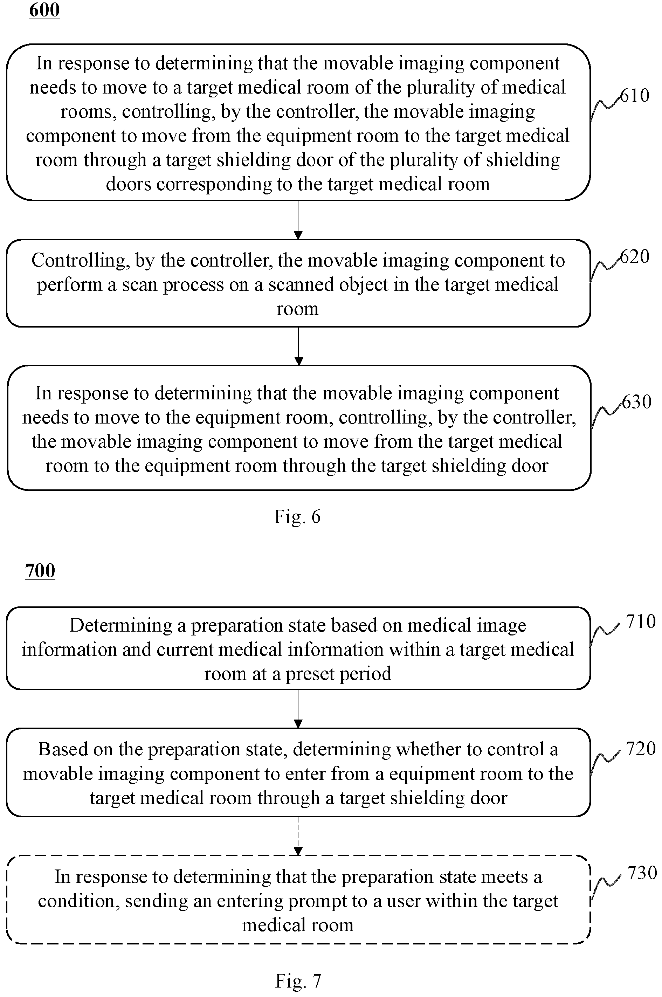

In some embodiments, the controller 130 is configured to control the movement of the movable imaging component 111 in response to the need for the movable imaging component 111 to move to the target medical room in the plurality of medical rooms 170, such that the movable imaging component 111 enters the target medical room from the equipment room 160 through the target shielding door corresponding to the target medical room in the plurality of shielding doors 120; control the movable imaging component 111 to perform an imaging process on a scanned object in the target medical room; and in response to the need for the movable imaging component 111 to move to the equipment room 160, control the movement of the movable imaging component 111 such that the movable imaging component 111 enters the equipment room 160 from the target medical room through the target shielding door. See the related descriptions of FIG. 6 to FIG. 7 for more.

In some embodiments, the controller 130 includes a plurality of control units, and each of the plurality of control units is provided in one of the plurality of medical rooms 170. In some embodiments, the control unit may be a microcontroller unit (MCU) chip, a dedicated energy management strategy (EMS) chip, a field-programmable gate array (FPGA), a central processing unit (CPU), or a graphics processing unit (GPU).

The control unit is the basic unit that implements the control function. In some embodiments, one control unit is provided in a medical room 170. As shown in FIG. 1, a first control unit 131 is provided in the first medical room 171 and a second control unit 132 is provided in the second medical room 172.

In some embodiments, the control unit may be provided as a stand-alone hardware device in the medical room 170. In other embodiments, the control unit may be integrally provided on other devices within the medical room 170, such as integrally provided on the medical bed 140 or a display 150. More on the medical bed 140 and the display 150 can be found in FIG. 4 and its related description.

When performing medical activities (e.g., hybrid operation), the doctor needs to perform corresponding operations in the medical room 170, and each medical room 170 is equipped with a control unit that allows the doctor to control, through the control unit, the movement of the movable imaging component 111 (e.g., between the corresponding medical room 170 and the equipment room 160, and/or in the corresponding medical room 170), movement of the medical bed 140 in the medical room, the opening and closing of the plurality of shielding doors 120, and the radiation exposure of the movable imaging component 111, or the like. In an application scenario, the doctor enters a corresponding medical room 170, obtains control of the control unit within the medical room 170, and thereby performs the corresponding control work. For example, the control unit may be provided as a stand-alone button device in a medical room 170. The button device includes a plurality of buttons each of which is configured to control a shielding door to be open or closed. A doctor enters the medical room 170, obtains control of the button device, and press the buttons of the button device to control the shielding door corresponding to the medical device to be open and control other shielding doors to be closed. As another example, the control unit may be integrally provided on a display 150 in a medical room 170. A doctor enters the medical room 170, obtains control of the display 150, and control, through a control interface on the display 150, the shielding door corresponding to the medical device to be open and control other shielding doors to be closed.

In some embodiments, for one of the plurality of control units, the control unit is activated to work after a predetermined password in the control unit is matched with an entered password.

In some embodiments, the control unit has a password module, and the doctor is required to enter a password when using the control unit, and when the entered password matches the predetermined password in the password module, the control unit may be activated to work, and the doctor may take over the control unit to perform the corresponding work.

In some embodiments, the above password is a character password. For example, the character password includes one or a combination of at least two of a numeric password, an alphabetic password, and a pattern password.

In some embodiments, the password module is configured based on biometrics. For example, a fingerprint recognition unit is provided in the password module, and after the user's fingerprint matches the pre-recorded fingerprint information in the fingerprint recognition unit, the user may activate the control unit to obtain control of the control unit. As another example, a face recognition unit is provided in the password module, and after the user's face is matched with the face information pre-recorded in the face recognition unit, the user may activate the control unit to obtain the control of the control unit. As still another example, a voiceprint recognition unit is provided in the password module, and after the user's voiceprint matches the pre-recorded voiceprint feature information (acoustic spectrum information) in the voiceprint recognition unit, the user may activate the control unit to obtain the control of the control unit. Understandably, in order to further enhance the security of the device, the above password method may also be in the form of a combination of at least two types of passwords, for example, a numeric password and a fingerprint password. When the user's fingerprint matches the pre-recorded fingerprint information, the user enters the password input interface of the control unit, and then the user can activate the control unit only after entering the correct password. Other combination of passwords can be similarly referred to the combination of numeric passwords and fingerprint passwords, and will not be explained here.

In some embodiments of the present disclosure, password matching can enhance the security of the device and avoid the control unit being triggered by mistake.

In some embodiments, the controller 130 is further configured to control the movable imaging component 111 to perform an imaging process or to stop an imaging process, and there is no need to provide a separate controller for the movable imaging component, which further saves costs.

In some embodiments, the controller 130 may also control work parameters of the movable imaging component 111. Details regarding how to control the work parameters can be found elsewhere in the present disclosure (e.g., descriptions in connection with FIG. 6).

In some embodiments, the medical system 100 further includes a plurality of medical beds 140. For each of the plurality of medical beds 140, the controller 130 is configured to plan a movement trajectory of the medical bed 140 based on environment information within the medical room 170, and to control the medical bed 140 to move based on the movement trajectory. In some embodiments, each of the medical rooms 170 may be provided with a medical bed. For example, as shown in FIG. 1, a first medical bed 141 corresponds to the first medical room 171, and a second medical bed 142 corresponds to the second medical room 172.

In some embodiments, the medical bed 140 may rotate, move in a straight and/or curved line on the floor, or move up and down in the direction of gravity, or the like. Rotation of the medical bed 140 includes rotation of the medical bed 140 around the vertical direction.

As an example, the medical bed 140 is parallel to the horizontal plane and is capable of horizontal linear motion, rotational motion around the vertical direction, elevating and/or falling motion in the direction of gravity. As shown in FIG. 1, the linear motion of the medical bed 140 is represented by the linear movement trajectory A in FIG. 1, the rotational motion of the end of the medical bed 140 near the imaging device 110 is represented by the rotational movement trajectory B1 in FIG. 1, and the rotational motion of theend of the medical bed 140 away from the imaging device 110 is represented by the rotational movement trajectory B2 in FIG. 1. As a carrier for carrying the patient, the medical bed 140 is movable within the medical room 170, which can support more clinical scenarios.

In some embodiments, the movement trajectory of the target medical bed includes the movement speed, the movement direction, the movement time, the movement distance, or the like of the target medical bed. Since the movement of the medical bed is in the form of movement (e.g., linear and/or curved movement) on the horizontal plane (floor) , rotation, and elevating and falling along the vertical direction, the movement speed of the medical bed includes the movement speed on the horizontal plane (floor), the rotation speed, and the elevating and falling speed, and the movement direction of the medical bed includes the movement direction on the horizontal plane (floor), the rotation direction, and the elevating and falling direction.

In some embodiments of the present disclosure, the controller 130 plans the movement trajectory of the medical bed 140 and controls the movement of the medical bed 140 based on the movement trajectory without manually moving the medical bed, which saves time and energy and avoids collision.

In some embodiments, the medical system 100 further includes a plurality of displays 150. For each of the plurality of displays 150, the display 150 is communicated with the controller 130 (e.g., the control unit) in the corresponding medical room 170 and the movable imaging component 111. In some embodiments, each of the plurality of medical rooms 170 is provided with a display 150. For example, as shown in FIG. 1, a first display 151 corresponds to the first medical room 171, and a second display 152 corresponds to the second medical room 172.

The display 150 is configured to display information related to the medical activities. For example, the display 150 includes a liquid crystal display, a cathode ray tube display, or the like.

The information displayed by the display 150 includes, but is not limited to, medical image information of the patient captured by the movable imaging component 111, patient information, movement trajectory of the movable imaging component 111 (including the movement trajectory of the movable imaging component 111 transferring to the medical room 170 and/or angular information in the rotational trajectory of the movable imaging component 111 around the medical bed 140), the movement trajectory of the medical bed 140, alarm prompt information that may be generated when the medical bed 140 moves based on the planned movement trajectory, the operation status of the movable imaging component 111 (e.g., dosage parameter of the imaging process , time of the imaging process, or the like), the angle of the individual movable joints in the robotic arm 113-1, movement prompt, physiological indicators of the patient (e.g., the oxygen concentration of the blood, the electrocardiographic signals, or the like), or the like. See FIG. 6 and its related instructions for more on the patient information, the movement trajectory of the movable imaging component 111, and the movement trajectory of the medical bed 140, and see FIG. 7 and its related instructions for more on the movement prompt.

In some embodiments of the present disclosure, displaying information related to a medical procedure by the display helps guide the conduct of the medical activity (e.g., operation) to improve the success rate of the medical activity (e.g., operation).

In some embodiments of the present disclosure, the medical system enables a single imaging device to be shared by a plurality of medical rooms without having to install a single imaging device in each of the medical rooms, thereby effectively increasing the utilization rate of the imaging device and greatly saving the equipment costs.

FIG. 6 is a flowchart of an exemplary process for controlling a medical system according to some embodiments of the present disclosure. As shown in FIG. 6, a process 600 includes the following steps. In some embodiments, the process 600 may be applied to a medical system (e.g., the medical system 100). In some embodiments, the process 600 is executed by a controller (e.g., the controller 130).

In some embodiments, the method for controlling a medical system is used to control a medical system. The medical system includes an equipment room, a plurality of medical rooms, an imaging device, a plurality of shielding doors, and a controller. The imaging device includes a movable imaging component. The movable imaging component is shared by the plurality of medical rooms, and the movable imaging component is configured to move between the equipment room and the plurality of medical rooms. For each of the plurality of shielding doors, the shielding door is provided between one of the plurality of medical rooms and the equipment room. The method comprises: in response to determining that the movable imaging component needs to move to a target medical room of the plurality of medical rooms, controlling, by the controller, the movable imaging component to move from the equipment room to the target medical room through a target shielding door of the plurality of shielding doors corresponding to the target medical room; controlling, by the controller, the movable imaging component to perform a scan process on an object in the target medical room; and in response to determining that the movable imaging component needs to move to the equipment room, controlling, by the controller, the movable imaging component to move from the target medical room to the equipment room through the target shielding door.

In step 610, in response to determining that the movable imaging component needs to move to a target medical room of the plurality of medical rooms, the controller controls the movable imaging component to move from the equipment room to the target medical room through a target shielding door of the plurality of shielding doors corresponding to the target medical room.

For more on the target medical room and the target shielding door, see the related descriptions above.

In some embodiments, when a scanned object in the target medical room needs to be imaged, the movable imaging component needs to move to the target medical room. The target shielding door is opened and the rest of the plurality of shielding doors is closed manually or by the controller, and the movable imaging component is controlled to move by the controller, so that the movable imaging component enters, through the target shielding door, from the equipment room to the target medical room to capture a medical image of the scanned object.

In some embodiments, the controller may also plan a movement trajectory of the imaging device and/or the target medical bed, control the imaging device and/or the target medical bed to move based on the movement trajectory so that a relative position between the movable imaging component and the target medical bed satisfies an imaging scan condition. In some embodiments, the controller may plan the movement trajectory of the imaging device and/or the target medical bed based on environment information within the target medical room.

The target medical bed is the medical bed in the target medical room.

In some embodiments, the environment information includes locations, shapes, or the like of people and items in the target medical room. In some embodiments, an image acquisition device is provided within the medical room, and the controller acquires the environment information within the medical room from the image acquisition device. The image acquisition device is used to acquire an image of the target medical room and send the image to the controller, and the controller determines the environment information based on the image of the target medical room. The image acquisition device may be at least one of a camera, a video camera, or the like.

The imaging scan condition is the condition that needs to be met to successfully acquire a medical image of a scanned subject. For example, the imaging scan condition includes that at least a portion of the medical bed is located in the imaging field of view (FOV) of the movable imaging component (e.g., the scanned subject is located in the medical bed and a region of interest (ROI) of the scanned subject is located in the imaging field of view of the movable imaging component), such that the movable imaging component can expose the scanned subject in the medical bed to the radiation, thereby acquiring the medical image of the scanned subject.

In some embodiments, the movement trajectory includes a movement trajectory of the imaging device, and/or a movement trajectory of the target medical bed. More about the movement trajectory of the imaging device and the movement trajectory of the target medical bed can be found above.

In some embodiments, the controller determines the movement trajectory of the imaging device and/or the target medical bed based on the environment information via a path planning algorithm. The path planning algorithm includes a Dijkstra algorithm, an A* algorithm, a D* algorithm, or the like. In some embodiments, the controller may also dynamically update the movement trajectory of the movable imaging component and/or the target medical bed during movement to make the movement trajectory of the imaging device and/or the target medical bed more consistent with the current environment information. The controller replans the movement trajectory by the path planning algorithm at a preset time interval. The operator can pre-set the preset time interval based on experience. For example, during the movement of the imaging device and/or the target medical bed, there may be a medical worker walking in the target medical room, and the controller may dynamically update the movement trajectory of the imaging device and/or the target medical bed so that the movement trajectory of the imaging device and/or the target medical bed avoids the medical worker.

In some embodiments, the environment information is visualized via a display, and an operator determines the movement trajectory of the movable imaging component and/or the target medical bed based on the visualized environment information.

In some embodiments, when the movement trajectory of the imaging device is determined based on the path planning algorithm, the controller controls the movement of the imaging device based on the movement trajectory of the imaging device, such that the movable imaging component arrives at the designated location. The designated location may be a fixed location or a location designated by a doctor. Understandably, the control of the movement of the imaging device may be achieved by adjusting the joint parameters of the respective mechanical joints of the robotic arm at the corresponding time points, and/or the movement speed, movement direction, movement time, and movement distance of the base movement.

In some embodiments, when the movement trajectory of the target medical bed is determined based on the path planning algorithm, the controller controls the movement of the target medical bed based on the movement trajectory of the target medical bed so that the target medical bed arrives at the designated location.

In some embodiments, when the movement trajectory of the imaging device and/or the target medical bed is determined based on the operator, the operator manually controls the movement of the imaging device and/or the target medical bed by means of the controller provided with components such as a control handle, a control joystick, or a control button, thereby causing the movable imaging component and/or target medical bed to reach the designated location.

In some embodiments, during movement of the target medical bed, the controller outputs an alarm prompt signal when a distance between the target medical bed and a first object is less than a first distance threshold. The doctor may choose to manually control the movement of the target medical bed via the controller, update the movement trajectory of the target medical bed, or stop the movement of the target medical bed via the controller after receiving the alarm prompt signal to avoid collisions between the target medical bed and the first object. The first object is an object that may collide with the target medical bed. The first object includes a medical device within the medical room, a wall, the shielding door, the imaging device (e.g., the movable imaging component and/or the base), the medical worker, or the like. The collisions include, but are not limited to, a collision between the target medical bed and a medical device within the medical room, a collision between the target medical bed and the walls of the medical room, a collision between the target medical bed and the shielding door, a collision between the imaging device (e.g., the movable imaging component and/or the base) and the target medical bed, and a collision between the medical worker and the target medical bed.

In some embodiments, the controller determines the distance between the target medical bed and the first object in a plurality of ways. For example, a plurality of distance sensors are provided at intervals on the target medical bed, and the controller may obtain the distance between the target medical bed and the first object based on the distance sensors. For another example, the controller collects the environment information within the medical room in real time through the image acquisition device, and determines the distance between the target medical bed and the first object through a proportional scale between the image and the actual space.

In some embodiments, the controller outputs an alarm prompt signal when a distance between the imaging device (e.g., the movable imaging component and/or the base) and a second object is less than a second distance threshold during movement of the imaging device. The doctor may choose to manually control the movement of the imaging device via the controller, update the movement trajectory of the imaging device, or stop the movement of the imaging device via the controller after receiving the alarm prompt signal, in order to avoid collisions between the imaging device and the second object. The second object refers to an object that may collide with the imaging device, and the second object includes a medical device within the medical room, the wall, the shielding door, the target medical bed, the medical worker, or the like. The collision includes, but is not limited to, a collision between the imaging device and the medical device within the medical room, a collision between the imaging device and the wall of the medical room, a collision between the imaging device and the shielding door, a collision between the imaging device and the target medical bed, and a collision between the imaging device and the medical worker.

In some embodiments, the controller determines the distance between the imaging device and the second object in a plurality of ways. For example, the distance between the movable imaging component and the second object is determined by providing distance sensors on the base and/or the movable imaging component or by providing the image acquisition device within the medical room. The method of determining the distance between the movable imaging component and the second object by providing the distance sensors or the image acquisition device is similar to the method of determining the distance between the target medical bed and the first object by providing the distance sensors or the image acquisition device described above. For example, the plurality of degrees of freedom of the robotic arm is realized based on the mechanical structure of the robotic arm itself. The robotic arm includes a plurality of sequentially connected mechanical joints, and two adjacent mechanical joints are rotationally connected. The spatial position of the movable imaging component in the spatial range can be obtained in real time based on the conversion of the joint parameters of the respective mechanical joints (including the joint angles), so that the distance between the movable imaging component and the second object may be determined based on the spatial position of the movable imaging component and the spatial position of the second object.

The first distance threshold and the second distance threshold may be a system default or set manually, and the alarm prompt signal includes, but are not limited to, voice prompts, image prompts, or the like.

In some embodiments, during relative movement generated by the medical bed and the imaging device (e.g., the movable imaging component and/or the base), the controller plans and updates, in real time, respective movement trajectories of the medical bed and the imaging device based on the environment information within the medical room, and controls the movements of the medical bed and the imaging device based on their respective planned movement trajectories in order to avoid the collisions. In some embodiments, when the relative position between the movable imaging component and the target medical bed meets the imaging scan condition, driven by the robotic arm, the controller drives a C-arm to rotate in a circumferential direction of the C-arm (e.g., rotate around the Z axis in FIG. 2) and/or rotate around the X axis in FIG. 2, in conjunction with the radiation source and the detector, to perform DSA imaging.

In some embodiments, when use of the imaging device is complete, the controller may control the target medical bed and/or the movable imaging component to return to an initial position by a method similar to the method described above, as can be seen in the description above.

In some embodiments, the controller causes the movable imaging component to arrive at the designated position in a corresponding medical room via a one-touch positioning system. The one-touch positioning system is a system with a robotic arm path-planning function, eliminating the need for manual adjustment of the motion parameters for each joint axis of the robotic arm, as the algorithm automatically calculates the motion parameters of the robotic arm and controls the robotic arm.

In some embodiments of the present disclosure, by controlling the movement of the movable imaging component and the target medical bed to move based on a movement trajectory, collisions resulting in damage to the device can be avoided, and the relative position between the movable imaging component and the target medical bed can be made to meet the imaging scan condition, thereby ensuring imaging quality of the movable imaging component.

In some embodiments, the controller may determine work information of the imaging device based on medical scheduling information, and control the movement of the movable imaging component between the equipment room and the plurality of medical rooms based on the work information.

The medical scheduling information is information related to performing medical activities. The medical scheduling information includes medical information for each medical activity. In some embodiments, the medical information includes starting time for the medical activity (e.g., time of operation, time of examination, etc.), medical duration of the medical activity (e.g., duration of operation, duration of examination, etc.), medical room of the medical activity, medical name of the medical activity (e.g., name of surgery, name of examination, etc.), prior diagnosis (diagnosis before the medical activity), patient information, whether the imaging device is required in the medical activity, or the like. The patient information includes at least one of the patient's name, gender, age, and medical history. In some embodiments, the controller obtains the medical scheduling information by accessing backend data of the hospital.

The work information of the imaging device is information related to a working schedule of the imaging device. In some embodiments, the work information of the imaging device includes a service time of the imaging device for the medical activity, the target medical room for the medical activity, initial work parameters of the imaging device for the medical activity, or the like.

The service time of the imaging device is the time when the imaging device needs to be used. The target medical room is the medical room where the imaging device is needed. More about the target medical room can be found above. In some embodiments, the controller determines the service time of the imaging device and the corresponding target medical room based on the medical scheduling information. For example, if the imaging device is required for a particular medical activity, the controller determines the medical time and medical room corresponding to the medical activity as the service time of the imaging device and the corresponding target medical room, respectively.

The initial work parameters are work parameters of the imaging device when the imaging device is in a standby mode. In some embodiments, the initial work parameters include a dose of the radiation exposure, a time of the radiation exposure, or the like.

In some embodiments, when the imaging device is a DSA device, the initial work parameters of the DSA device are the initial parameters displayed to users when the DSA device arrives at the corresponding medical room after users obtain control of the DSA device. Users can adjust the initial work parameters according to medical needs to obtain formal work parameters of the imaging device, instead of directly setting the formal work parameters without any reference, which can save time.

In some embodiments, the controller may determine the initial work parameters based on the medical name, the prior diagnosis, and the patient information by querying a first preset table.

The first preset table includes a correspondence of the medical name, the prior diagnosis, and the patient information with the initial work parameters. The first preset table may be obtained by constructing a table based on the work parameters actually used by the imaging device, and the corresponding medical name, prior diagnosis, and patient information in the historical data.

In some embodiments, the controller may determine the initial work parameters based on the medical name, the prior diagnosis, and/or the patient information through a parameter recommendation model.

The parameter recommendation model is a model used to determine the initial work parameters. In some embodiments, the parameter recommendation model is a machine learning model, e.g., a Neural Network (NN) model, a Deep Neural Networks (DNN) model, or the like.

In some embodiments, the input to the parameter recommendation model includes the medical name, the prior diagnosis, and/or the patient information, and the output of the parameter recommendation model includes the initial work parameters.

In some embodiments, the parameter recommendation model is obtained based on a plurality of first training samples with first labels. For example, the controller may input the plurality of first training samples into an initial parameter recommendation model, construct a first loss function based on the outputs of the initial parameter recommendation model and the first labels, iteratively update parameters of the initial parameter recommendation model, and when an iteration termination condition is satisfied, terminate the iteration to obtain the trained parameter recommendation model. The method of iteratively updating includes, but is not limited to, a gradient descent method, and the iteration termination condition may be the convergence of the first loss function or the number of iterations reaching a threshold.

The first training sample includes a sample medical name, a sample prior diagnosis, and/or sample patient information, and the first training sample may be obtained based on historical data. The first labels are actual work parameters corresponding to the first training samples.

In some embodiments of the present disclosure, the initial work parameters are determined by the parameter recommendation model, which can make the initial work parameters more accurate and reduce the user's manual adjustment.

In some embodiments of the present disclosure, determining the work information of the imaging device based on the medical scheduling information and controlling the imaging device to work based on the work information of the imaging device are able to automate the control process without excessive human intervention.

In some embodiments, the controller may determine movement information of the imaging device based on the work information and location distribution information of the imaging device, and control the imaging device to perform the movement based on the movement information. Planning for the movement information may be performed in advance because the number and arrangement of the plurality of medical rooms in the medical system can be varied, and the movement methods may be used in combination.

The location distribution information refers to location distribution of the work environment. In some embodiments, the location distribution information includes locations of the medical rooms, locations of the shielding doors, a location of the track, an initial parking location of the imaging device, or the like.

The movement information is information related to the movement of the imaging device. In some embodiments, the movement information includes a parking location, a movement mode, a movement path (which may also be referred to as the movement trajectory), or the like.

In some embodiments, the parking location of the imaging device refers to a location where the movable imaging component and/or the base parks after movement. For example, when the base is not fixed within the equipment room, the parking location of the imaging device includes a parking location of the end of the robotic arm and a parking location of the base. For another example, when the base is fixed within the equipment room, the parking location of the imaging device is the parking location of the end of the robotic arm.

When the imaging device is in work, the imaging device is in the initial parking location, which is located in the equipment room (e.g., the imaging device is located in the center of the equipment room with the robotic arm in the fold state). When the imaging device is working, the parking location of the imaging device is a location set in the target medical room.

In some embodiments, the movement mode corresponds to a moveable device when the base is not fixed within the equipment room. When the movable device is wheels, the movement mode is through the wheels; and when the movable device is a track connecting device, the movement mode is along the track.

In some embodiments, when the base is fixed within the equipment room, the movement is by rotation of the movable imaging component and by extension and folding of the robotic arm.

The movement path is the route traveled by the movable imaging component and/or the base.

In some embodiments, the controller may determine the movement path by a path planning algorithm based on the work information and the location distribution information of the imaging device. For example, for the work information of the imaging device in a period of time in the future (e.g., tomorrow), the controller obtains work time of the imaging device and corresponding target medical rooms based on the work information of the imaging device, and then obtains the parking location corresponding to each target medical room. The controller determines, through the path planning algorithm, a movement path from the initial parking location to a first parking location in the first target medical room, and a movement path from the first parking location to the initial parking location; the controller determines, through the path planning algorithm, a movement path from the initial parking location to a second parking location in the second target medical room, and a movement path from the second parking location to the initial parking location;... the controller determines, through the path planning algorithm, a moving path from the initial parking location to an nth parking location in the nth target medical room, and a moving path from the nth parking location to the initial parking location, thereby obtaining a complete movement path of the imaging device for the work information. The first parking location, the second parking location, ......, and the nth parking location are the locations at which the imaging device needs to park in time sequence based on the work information of the imaging device. In some embodiments, according to the work information (e.g., the service time of the imaging device), the controller may determine a departure time from the initial parking location to each parking location. When the departure time is reached, the imaging device may automatically move from the initial parking location to the corresponding target medical room along the corresponding movement path. For example, the controller may determine a time that is 30 minutes before the service time of the imaging device in a target medical room as the departure time from the initial parking location to the parking location in the target medical room. In this way, the imaging device can automatically start to move from the initial parking location in advance, thereby realizing the automatic movement control of the imaging device and ensuring that the movable imaging component arrives at the target medical room in time.

In some embodiments, the controller determines drive parameters for the movable device based on the movement information to drive the movable device to drive the imaging device.

In some embodiments of the present disclosure, determining the movement information through the path planning algorithm and controlling the movement of the imaging device based on the movement information enable the imaging device to move flexibly based on an optimal path so as to save time of the movement of the imaging device.

In some embodiments, the controller may determine a preparation state of the target medical room, and in response to determining that the preparation state is suitable for the imaging device to enter the target medical room, the controller may send an movement prompt to the user in the target medical room, and in response to receiving the user's confirmation of the movement prompt, the controller may control the movable imaging component to enter the target medical room from the equipment room through the open target shielding door. For more on the preparation state and the movement prompt, see the descriptions in connection with FIG. 7.

In step 620, the controller may control the movable imaging component to perform a scan process on a scanned object in the target medical room.

In some embodiments, the scan process includes an imaging process and stopping the imaging process. See the related description above for more on the imaging process and stopping the imaging process.

In step 630, in response to determining that the movable imaging component needs to move to the equipment room, the controller may control the movable imaging component to move from the target medical room to the equipment room through the open target shielding door.

In some embodiments, when the imaging of the scanned object is completed, the movable imaging component needs to move to the equipment room, and the movement of the movable imaging component is controlled by the controller such that the movable imaging component enters, through the target shielding door, from the target medical room to the equipment room.

In some embodiments of the present disclosure, controlling the movement of the movable imaging component and the scan process by the controller can enable the movable imaging component to conveniently move between the equipment room and the plurality of medical rooms and perform the scan process of the scanned object in the target medical room.

It should be noted that the foregoing description of the process 600 is intended to be exemplary and illustrative only, and does not limit the scope of application of this disclosure. For those skilled in the art, various corrections and changes can be made to the process 600 under the guidance of this disclosure. However, these corrections and changes remain within the scope of the present disclosure.

In some embodiments, the controller determines a maintenance time for the imaging device based on historical movement information, historical work information, and historical work environment information of the imaging device. When the maintenance time is reached, the controller controls the imaging device to enter a maintenance state.

The historical movement information is movement information of the imaging device during the time period from the last maintenance to the current moment. The historical movement information includes a historical number (count) of movements of the imaging device, a historical distance of movement, or the like. The controller may read the historical movement information directly from a storage device. See descriptions in connection with FIG. 6 for more on the movement information of the imaging device.

The historical work environment information is work environment information during the time period from the last maintenance to the current moment. The work environment information includes temperature, humidity, or the like, of the environment in which the imaging device is located every day. The controller may obtain the temperature and humidity by a temperature sensor and a humidity sensor installed in the equipment room and the medical room, respectively.

In some embodiments, the temperature and humidity of the environment in which the imaging device is located are constantly changing throughout the day. The controller may use an average of a plurality of temperatures during a day as the temperature of the environment in which the imaging device is located during the day, and an average of a plurality of humidities during the day as the humidity of the environment in which the imaging device is located during the day. The controller may also use the temperature with the longest duration within the day as the temperature of the environment in which the imaging device is located during the day, and the humidity with the longest duration within the day as the humidity of the environment in which the imaging device is located during the day.

In some embodiments, the controller may determine the maintenance time by querying a second preset table based on the historical movement information, the historical work information, and the historical work environment information.

The second preset table includes a correspondence between the maintenance time and the historical movement information, the historical work information, and the historical work environment information, and the second preset table may be constructed based on historical data.

In some embodiments, the controller may predict the maintenance time based on the historical movement information, the historical work information, and/or the historical work environment information through a prediction model.

The prediction model is a model for predicting the maintenance time, and in some embodiments, the prediction model is a machine learning model, e.g., the prediction model is a Neural Network (NN) model, or the like.

The input to the prediction model includes historical movement information, historical work information, and historical work environment information, and the output of the prediction model includes the maintenance time.

In some embodiments, the controller may obtain the prediction model based on a plurality of second training samples with second labels. For example, the controller may input the plurality of second training samples into an initial prediction model, construct a second loss function based on the outputs of the initial prediction model and the second labels, iteratively update, based on the second loss function, parameters of the initial prediction model, and when an iteration termination condition is satisfied, terminate the iteration to obtain the trained prediction model. The method of iteratively updating includes, but is not limited to, a gradient descent method, and the iteration termination condition may be the convergence of the second loss function or the number of iterations reaching a threshold.

The second training sample includes sample movement information, sample work information, and/or sample work environment information of a sample imaging device, and the second training sample may be obtained based on historical data of the sample imaging device. The second label is a time interval between the corresponding moment of the second training sample and the next maintenance of the sample imaging device. In some embodiments, for a second training sample of a sample imaging device, a device inspection is performed on the sample imaging device at a first history time (the corresponding moment of the second training sample), a second history time, a third history time, ......, and an nth history time to obtain a first inspection result, a second inspection result, a third inspection result,......, and an nth inspection result, respectively. The second history time, the third history time, ......, and the nth history time are after the first history time. Among the n history time, the earliest history time corresponding to which the inspection result indicates a significant deterioration of a device state of the sample imaging device is designated as a target history time. An interval between the first history time and the target history time may be used as the second label corresponding to the second training sample. The device state refers to a health state of the sample imaging device. For example, the device state includes Central Processing Unit (CPU) utilization, memory usage, operating temperature, remaining life, or the like. When a difference between the device state at the first history time and the device state at a history time after the first history time is larger than a health state threshold, it is indicated a significant deterioration of the device state of the sample imaging device at the history time. The time interval between two adjacent history time of the first history time, the second history time, the third history time, ......, and the nth history time is less than a first time threshold. The first time threshold may be preset.

In some embodiments, the input to the prediction model also includes a future work frequency.

The future work frequency is a number (count) of times the imaging device will work in a preset time period in the future. The controller may determine the future work frequency based on historical data of the imaging device.

In some embodiments, the controller may determine the future work frequency based on the current time by querying a third preset table.

The third preset table includes a correspondence between the current time and the work frequency, and the third preset table may be constructed based on historical data of the imaging device.

In some embodiments, when the input to the prediction model includes the future work frequency, the second training sample further includes a sample future work frequency of a sample imaging device, and the sample future work frequency may be obtained based on historical data of the sample imaging device.

Some diseases are more prevalent at specific times of the year, for example, during the winter months when the probability of cardiovascular disease is higher, the number of cardiovascular operations and/or examinations that need to be performed increases, and the work frequency of the imaging device increases. In order to ensure the proper functioning of the imaging device, it is necessary to maintain the imaging device in advance.

Some embodiments of the present disclosure enable the prediction model to accurately predict an appropriate time for the next maintenance, reducing negative impacts due to machine failure.

In some embodiments, the controller controls the imaging device to enter the maintenance state when the maintenance time is reached. In the maintenance state, the imaging device stops working. Until the maintenance of the imaging device is complete, the imaging device exits the maintenance state, and the imaging device can work normally.

More frequent maintenance is required due to increased utilization of the imaging device and the fact that the imaging device needs to be moved frequently. So consideration is given to determining the next maintenance time based on the usage of the imaging device.

Some embodiments of the present disclosure, where timely maintenance is carried out according to the usage of the imaging device, can effectively avoid malfunctioning of the imaging device.

FIG. 7 is a flowchart of an exemplary process for controlling a movable imaging component to move to a target medical room according to some embodiments of the present disclosure. As shown in FIG. 7, a process 700 includes the following steps. In some embodiments, the process 700 is performed by a controller.

In some embodiments, the controller determines a preparation state based on medical image information and current medical information within a target medical room at a preset period through a state prediction model, and based on the preparation state, determines whether to control the movable imaging component to enter from the equipment room to the target medical room through a target shielding door.

In some embodiments, in response to determining that the preparation state is suitable for the movable imaging component to enter the target medical room, the controller sends a movement prompt to the user in the target medical room, and in response to the user's confirmation of the movement prompt, controls the movable imaging component to enter the target medical room from the equipment room through the target shielding door.

In step 710, the controller determines a preparation state based on medical image information and/or current medical information within a target medical room at a preset period.

The medical image information is image information at the current time related to performing a medical activity. The controller may acquire the medical image information based on an image acquisition device. For more on the image acquisition device, see descriptions in connection with FIG. 6.

The current medical information is medical information corresponding to the target medical room. For more on the medical information, see descriptions in connection with FIG. 6.

In some embodiments, the preset period is a system default, e.g., the preset period is 3 minutes.

In some embodiments, the preset period is related to the current medical information. For example, the preset period is correlating to an amount of time that a current medical activity needs to be prepared, and the longer the amount of time that the current medical activity needs to be prepared, the longer the preset period.

In some embodiments, the controller may determine the preset period based on the amount of time that the current medical activity needs to be prepared by querying a fourth preset table.

The fourth preset table includes a correspondence between the amount of time that the current medical activity needs to be prepared and the preset period, and the fourth preset table can be constructed based on historical data.

In some embodiments of the present disclosure, determining the preset period based on the amount of time that the current medical activity needs to be prepared can appropriately increase the preset period to reduce the amount of data processed by the controller when the amount of time that the current medical activity needs to be prepared is long.

The preparation state reflects the readiness of the medical activity. The preparation state includes a state that it is suitable for the imaging device to enter the target medical room and a state that it is unsuitable for the imaging device to enter the target medical room.

In some embodiments, the controller may determine the preparation state based on the medical image information and the current medical information through a state prediction model.

Understandably, based on the medical image information, a determination may be made as to whether the medical worker is ready to deal with the entering of the DSA device. For example, when the medical worker is dressed in a protection suit and other related medical devices (e.g., the display, the controller, tools of surgery, etc.) are ready for work, it is indicated that it is suitable for the imaging device to enter the target medical room. But different medical activities have different conditions that need to be met, for example, for medical activity A, the DSA device may enter the target medical room as long as the user is dressed in the protection suit; for medical activity B, the DSA device may enter the target medical room when the user is dressed in the protection suit, a medical bed is located in place, and the other related medical devices (e.g., the display, the controller, tools of surgery, etc.) are ready for work. Therefore, current medical information needs to be considered when determining whether or not it is appropriate for the imaging device to enter the target medical device.

The state prediction model is a model used to determine the preparation state. In some embodiments, the state prediction model is a machine learning model, e.g., a Deep Neural Networks (DNN) model, or the like.

In some embodiments, the input to the state prediction model includes the medical image information and/or the current medical information, and the output of the state prediction model includes the preparation state. In some embodiments, the preparation state is represented by a number, with 0 indicating that it is unsuitable for the imaging device to enter the target medical room and 1 indicating that it is suitable for the imaging device to enter the target medical room.

In some embodiments, the state prediction model is obtained based on a plurality of third training samples with third labels. For example, the controller may input the plurality of third training samples into an initial state prediction model, construct a third loss function based on the outputs of the initial state prediction model and the third labels, iteratively update, based on the third loss function, parameters of the initial state prediction model, and when the iteration termination condition is satisfied, terminate the iteration to obtain the trained state prediction model. The methods of iterative updating include, but are not limited to, gradient descent, and the iteration termination condition may be the convergence of the third loss function or the number of iterations reaching a threshold.

The third training sample includes sample medical image information and sample medical information, and the third training sample may be obtained based on historical data. The third label is the preparation state corresponding to the third training sample. In some embodiments, for a set of third training samples, if the moment when the user controlled the imaging device to enter the target medical room is after the moment of acquisition of the sample image information, and the time interval between the two is less than a second time threshold, the third label is set to 1. If the moment when the user controlled the imaging device to enter the target medical room is after the moment of acquisition of the sample image information, and the time interval between the two is more than the second time threshold, the third label is set to 0. The second time threshold may be preset.

In some embodiments, the state prediction model includes a feature extraction layer and a prediction layer.

The input of the feature extraction layer includes the medical image information and the output of the feature extraction layer includes medical image features.

The medical image features are features extracted from the medical image information.

The input to the prediction layer includes the medical image features and the current medical information, and the output of the prediction layer includes the preparation state.

In some embodiments, the feature extraction layer and the prediction layer may be obtained by joint training. The joint training sample includes the sample medical image information and the sample medical information, and the joint training label is the preparation state corresponding to the joint training sample. The sample medical image information may be input into an initial feature extraction layer to obtain sample medical image features output from the initial feature extraction layer. The sample medical image features and the sample medical information may be input into an initial prediction layer, and the preparation state output from the initial prediction layer is obtained. A joint loss function may be constructed based on the preparation state and the joint training labels, the parameters may be iteratively updated based on the joint loss function, and the trained feature extraction layer and the prediction layer may be obtained. In some embodiments, the joint training samples are obtained in the same manner as the third training samples, and the joint training labels are obtained in the same manner as the third labels.

Some embodiments of the present disclosure determine the preparation state by the state prediction model, and the preparation state obtained is more accurate.

In step 720, based on the preparation state, the controller may determine whether to control the movable imaging component to enter from the equipment room to the target medical room through the target shielding door.

In some embodiments, in response to determining that the preparation state meets a condition, the controller may send the movement prompt to the user within the target medical room. That is, with the process 700 may proceed to step 730. In response to determining that the preparation state does not meet the condition, the execution of the step 710 may be continued.

In step 730, in response to determining that the preparation state meets a condition, the controller may send an movement prompt to the user within the target medical room.

In some embodiments, the preparation state meeting the condition means that the preparation sate indicates that it is suitable for the imaging device to enter the target medical room.

The movement prompt is a prompt that reminds the user that the imaging device can enter the target medical room. The movement prompt may be in a variety of forms, e.g., the movement prompt includes a voice prompt, an image prompt, or the like.

In some embodiments, upon receiving the movement prompt, the user may confirm the movement prompt (e.g., by clicking a confirmation button on a display, or the like). In response to the user's confirmation of the movement prompt, the controller controls movement of the movable imaging component to enter the target medical room from the equipment room through the target shielding door.

In some embodiments of the present disclosure, determining the preparation state a plurality times at the preset period enables the imaging device to timely enter the target medical room to avoid interferes with the medical activity cause by the imaging device entering the target medical room too late.

Sending the movement prompt to the user after the preparation state indicates that it is suitable for the imaging device to enter the target medical room, and controlling the imaging device to enter the target medical room until the user confirms the movement prompt can avoid interferes with the medical activity caused by the imaging device entering the target medical room too early.

It should be noted that the foregoing description of the process 700 is intended to be exemplary and illustrative only and does not limit the scope of application of the present disclosure. For a person skilled in the art, various corrections and changes can be made to the process 700 under the guidance of this disclosure. However, these corrections and changes remain within the scope of the present disclosure. For example, the step 730 may be omitted. When the preparation state indicates that it is suitable for the imaging device to enter the target medical room, the controller directly controls movement of the movable imaging component from the equipment room to the target medical room through the target shielding door, without the confirmation of the user.

The workflow of controlling the medical system 100 shown in some embodiments of the present disclosure is illustrated using the first medical room 171 and the second medical room 172 shown in FIG. 1 as examples. The user enters the first medical room 171 and enters a corresponding password to obtain control of the first control unit 131 in the first medical room 171, for example, the user may obtain the control of the first control unit 131 only after completing voiceprint recognition and character password matching successively. The user opens the first shielding door 121 corresponding to the first medical room 171 via the first control unit 131. The user sends instructions to the imaging device 110 via the first control unit 131 to cause the movable imaging component 111 of the imaging device 110 to move through the opened first shielding door 121 to a designated location within the first medical room 171. The user keeps the second shielding door 122 corresponding to the second medical room 172 closed via the first control unit 131. The user activates the imaging device 110 via the first control unit 131, which can be understood as the corresponding function of the imaging device 110 within the first medical room 171 being activated, the corresponding function of the imaging device 110 within the second medical room 172 being deactivated, and controls the first medical bed 141 and the movable imaging component 111 to reach corresponding positions so as to perform a scan process to capture a medical image of the patient. While controlling the relative movement of the first medical bed 141 and the movable imaging component 111, the first control unit 131 plans in real time respective movement trajectories of the first medical bed 141 and the movable imaging component 111 to avoid an expected collision situation. The user can observe, in real time, the movement trajectories of the first medical bed 141 and the movable imaging component 111, as well as the captured image information, on the first display 151.

It should be noted that the devices within each of the medical rooms 170 are substantially the same, and after the user obtains control of the controller 130 within one of the medical rooms 170, the functions of the relative devices within the medical room 170 will be in an activated state and can be controlled to work by the controller 130, and the functions of the relative devices within the rest medical rooms 170 will be in an inactivated state. Exemplarily, the user obtains control of the first medical room 171, and the corresponding functions in the first medical room 171 are activated, such as information interaction functions (including information communication between devices, human-computer interaction, or the like), ray exposure functions (i.e., a ray exposure function of the DSA device), functions for controlling the movement of the devices (including controlling the movement of the C-arm, controlling the movement of the medical bed 140), prompting alarm functions (including an alarm for the radiation dose exceeding a preset amount, an anti-collision alarm during the movement of the devices, or the like), image display functions (the display displaying medical image information, movement trajectories of the devices, physiological parameters of the patient, or the like), or the like are activated, whereas the above-mentioned functions provided in the second medical room 172 are correspondingly inactivated, and when the user obtains the control of the second medical room 172, the above-mentioned functions of the second medical room 172 will be activated, and the above-mentioned functions of the first medical room 171 will be inactivated accordingly. That is, the user may realize the switching of control within each medical room 170 by completing a password match to activate the corresponding functions of the medical room 170.

Further, when the user obtains control of one of the medical rooms 170, the user may collect environment information of the medical room 170 through cameras installed at a plurality of locations within the medical room 170, so as to plan the movement trajectory of the C-arm based on the environment information, allowing the C-arm to enter the medical room 170 more flexibly, and automatically realizing the collision prevention requirement. As well as controlling the translation and rotation of the medical bed 140 based on the environment information, realizing the need for collision prevention of the medical bed 140 and the need for the location of the medical bed 140 during the scanning examination.

In some embodiments, the workflow of controlling the medical system 100 further includes: in response to the time interval between the current time and the service time of the imaging device 110 being less than a preset duration (e.g., 10min), the controller 130 sends a reminder to the user in the target medical room; upon confirmation of the reminder by the user, the controller 130 controls the target shielding door to open, and the imaging device 110 moves from an initial parking location to a target parking location in the medical room and activates the corresponding functions; the imaging device 110 sets work parameters to initial work parameters and displays them to the user; upon completion of the medical activity and confirmation by the user, the imaging device 110 closes the corresponding functions and returns to the initial parking location for sterilization and waiting.

Some embodiments of the present disclosure provide a non-transitory computer readable medium, comprising executable instructions, wherein when executed by one or more processors of a computing device, the executable instructions cause the computing device to perform a method for controlling a medical system, the medical system comprising: an equipment room and a plurality of medical rooms; an imaging device, the imaging device including a movable imaging component, the movable imaging component being shared by the plurality of medical rooms, and the movable imaging component being configured to move between the equipment room and the plurality of medical rooms; a plurality of shielding doors, for each of the plurality of shielding doors, the shielding door being provided between one of the plurality of medical rooms and the equipment room; and a controller; the method including: in response to determining that the movable imaging component needs to move to a target medical room of the plurality of medical rooms, controlling, by the controller, the movable imaging component to move from the equipment room to the target medical room through a target shielding door of the plurality of shielding doors corresponding to the target medical room; controlling, by the controller, the movable imaging component to perform a scan process on an object in the target medical room; and in response to determining that the movable imaging component needs to move to the equipment room, controlling, by the controller, the movable imaging component to move from the target medical room to the equipment room through the target shielding door.

## Claims

1. A medical system, comprising:
an equipment room (160) and a plurality of medical rooms (170);
an imaging device (110), the imaging device (110) including a movable imaging component (111), the movable imaging component (111) being shared by the plurality of medical rooms (170), and the movable imaging component (111) being configured to move between the equipment room (160) and the plurality of medical rooms (170); and
a plurality of shielding doors (120), for each of the plurality of shielding doors (120), the shielding door (120) being provided between one of the plurality of medical rooms (170) and the equipment room (160), wherein
the imaging device (110) includes a base (112) configured to support the movable imaging component (111),
the movable imaging component (111) is connected to the base (112) by means of a robotic arm (113-1) having a plurality of degrees of freedom,
the movable imaging component (111) is movable relative to the base (112); and
during movement of the movable imaging component (111) between the equipment room (160) and the plurality of medical rooms (170), the base (112) is located in the equipment room (160).

2. The medical system of claim 1, wherein the plurality of medical rooms (170) are arranged around the equipment room (160).

3. The medical system of claim 1 or claim 2, wherein when the movable imaging component (111) needs to move to a target medical room of the plurality of medical rooms (170),
a target shielding door of the plurality of shielding doors (120) between the target medical room and the equipment room (160) is configured to be open, and the rest of the plurality of shielding doors (120) are configured to be closed.

4. The medical system of claim 3, wherein the target medical room and the equipment room (160) form a sealed space.

5. The medical system of claim 3 or claim 4, wherein the rest of the plurality of medical rooms (170) is isolated from the target medical room and the equipment room (160).

6. The medical system of any one of claim 1-5, wherein the imaging device (110) is a floor-mounted digital subtraction angiography (DSA) device.

7. The medical system of any one of claims 1-6, wherein the base (112) is fixed within the equipment room (160).

8. The medical system of any one of claim 1-7, further comprising a controller (130), the controller (130) being configured to:
control the plurality of shielding doors (120) to open or close, or to control movement of the movable imaging component (111) between the equipment room (160) and the plurality of medical rooms (170); or
control the imaging device (110) to perform an imaging process or stop the imaging process.

9. The medical system of claim 8, further comprising a plurality of medical beds (140);
wherein for each of the plurality of medical beds (140), the controller (130) is configured to plan a movement trajectory of the medical bed (140) based on environment information within the medical room (170) where the medical bed (140) is located, and to control the medical bed (140) to move based on the movement trajectory.

10. The medical system of any one of claim 1-9, wherein at least one of the plurality of medical rooms (170) includes a hybrid operating room.

11. A method for controlling a medical system (100), the medical system (100) comprising:
an equipment room (160) and a plurality of medical rooms (170);
an imaging device (110), the imaging device (110) including a movable imaging component (111), the movable imaging component (111) being shared by the plurality of medical rooms (170), and the movable imaging component (111) being configured to move between the equipment room (160) and the plurality of medical rooms (170);
a plurality of shielding doors (120), for each of the plurality of shielding doors (120), the shielding door (120) being provided between one of the plurality of medical rooms (170) and the equipment room (160), wherein
the imaging device (110) includes a base (112) configured to support the movable imaging component (111),
the movable imaging component (111) is connected to the base (112) by means of a robotic arm (113-1) having a plurality of degrees of freedom,
the movable imaging component (111) is movable relative to the base (112); and
during movement of the movable imaging component (111) between the equipment room (160) and the plurality of medical rooms (170), the base (112) is located in the equipment room (160); and
a controller (130);
the method comprising:
in response to determining that the movable imaging component (111) needs to move to a target medical room of the plurality of medical rooms (170), controlling, by the controller (130), the movable imaging component (111) to move from the equipment room (160) to the target medical room through a target shielding door of the plurality of shielding doors (120) corresponding to the target medical room;
controlling, by the controller (130), the movable imaging component (111) to perform a scan process on an object in the target medical room; and
in response to determining that the movable imaging component (111) needs to move to the equipment room (160), controlling, by the controller (130), the movable imaging component (111) to move from the target medical room to the equipment room (160) through the target shielding door.

12. The method of claim 11, further comprising:
planning a movement trajectory of the movable imaging component (111) and/or a target medical bed, the target medical bed being disposed within the target medical room; and
controlling the movable imaging component (111) and/or the target medical bed to move based on the movement trajectory so that a relative position between the movable imaging component (111) and the target medical bed satisfies an imaging scan condition.

13. The method of claim 11 or claim 12, wherein the base (112) is fixed within the equipment room (160).

14. The method of any one of claims 11-13, wherein the plurality of medical rooms (170) are arranged around the equipment room (160).

15. A non-transitory computer readable medium, comprising executable instructions, wherein when executed by one or more processors of a computing device, the executable instructions cause the computing device to perform a method for controlling a medical system (100), the medical system (100) comprising:
an equipment room (160) and a plurality of medical rooms (170);
an imaging device (110), the imaging device (110) including a movable imaging component (111), the movable imaging component (111) being shared by the plurality of medical rooms (170), and the movable imaging component (111) being configured to move between the equipment room (160) and the plurality of medical rooms (170);
a plurality of shielding doors (120), for each of the plurality of shielding doors (120), the shielding door (120) being provided between one of the plurality of medical rooms (170) and the equipment room (160), wherein
the imaging device (110) includes a base (112) configured to support the movable imaging component (111),
the movable imaging component (111) is connected to the base (112) by means of a robotic arm (113-1) having a plurality of degrees of freedom,
the movable imaging component (111) is movable relative to the base (112); and
during movement of the movable imaging component (111) between the equipment room (160) and the plurality of medical rooms (170), the base (112) is located in the equipment room (160); and
a controller (130);
the method including:
in response to determining that the movable imaging component (111) needs to move to a target medical room of the plurality of medical rooms (170), controlling, by the controller (130), the movable imaging component (111) to move from the equipment room (160) to the target medical room through a target shielding door of the plurality of shielding doors (120) corresponding to the target medical room;
controlling, by the controller (130), the movable imaging component (111) to perform a scan process on an object in the target medical room; and
in response to determining that the movable imaging component (111) needs to move to the equipment room (160), controlling, by the controller (130), the movable imaging component (111) to move from the target medical room to the equipment room (160) through the target shielding door.

## Patentansprüche

1. Medizinisches System, umfassend:
einen Geräteraum (160) und eine Vielzahl von medizinischen Räumen (170);
eine Bildgebungsvorrichtung (110), wobei die Bildgebungsvorrichtung (110) eine bewegliche Bildgebungskomponente (111) einschließt, wobei die Bildgebungskomponente (111) von der Vielzahl von medizinischen Räumen (170) gemeinsam genutzt wird, und wobei die bewegliche Bildgebungskomponente (111) so konfiguriert ist, dass sie sich zwischen dem Geräteraum (160) und den medizinischen Räumen (170) bewegt; und
eine Vielzahl von Abschirmtüren (120) für jede der Vielzahl von Abschirmtüren (120), wobei die Abschirmtür (120) zwischen einem der Vielzahl von medizinischen Räumen (170) und dem Geräteraum (160) bereitgestellt ist, wobei
die Bildgebungsvorrichtung (110) einen Sockel (112) einschließt, der zum Tragen der beweglichen Bildgebungskomponente (111) konfiguriert ist,
die bewegliche Bildgebungskomponente (111) mittels eines Roboterarms (113-1), der eine Vielzahl von Freiheitsgraden aufweist, mit dem Sockel (112) verbunden ist,
die bewegliche Bildgebungskomponente (111) in Bezug auf den Sockel (112) bewegbar ist; und
während Bewegung der beweglichen Bildgebungskomponente (111) zwischen dem Geräteraum (160) und der Vielzahl von medizinischen Räumen (170) der Sockel (112) im Geräteraum (160) gelegen ist.

2. Medizinisches System nach Anspruch 1, wobei die Vielzahl von medizinischen Räumen (170) um den Geräteraum (160) angeordnet ist.

3. Medizinisches System nach Anspruch 1 oder Anspruch 2, wobei, wenn sich die bewegliche Bildgebungskomponente (111) in einen medizinischen Zielraum der Vielzahl von medizinischen Räumen (170) bewegen muss,
eine Zielabschirmungstür der Vielzahl von Abschirmungstüren (120) zwischen dem medizinischen Zielraum und dem Geräteraum (160) so konfiguriert ist, dass sie offen ist und die übrigen der Vielzahl von Abschirmungstüren (120) so konfiguriert sind, dass sie geschlossen sind.

4. Medizinisches System nach Anspruch 3, wobei der medizinische Zielraum und der Geräteraum (160) einen abgedichteten Raum bilden.

5. Medizinisches System nach Anspruch 3 oder Anspruch 4, wobei der Rest der Vielzahl von medizinischen Räumen (170) vom medizinischen Zielraum und dem Geräteraum (160) isoliert ist.

6. Medizinisches System nach einem der Ansprüche 1-5, wobei es sich bei der Bildgebungsvorrichtung (110) um eine bodenmontierte digitale Subtraktionsangiographievorrichtung (DSA) handelt.

7. Medizinisches System nach einem der Ansprüche 1-6, wobei der Sockel (112) innerhalb des Geräteraums (160) fixiert ist.

8. Medizinisches System nach einem der Ansprüche 1-7, weiter umfassend eine Steuereinheit (130), wobei die Steuereinheit (130) konfiguriert ist zum:
Steuern der Vielzahl von Abschirmungstüren (120) zum Öffnen oder Schließen oder Steuern von Bewegung der beweglichen Bildgebungskomponente (111) zwischen dem Geräteraum (160) und der Vielzahl von medizinischen Räumen (170); oder
Steuern der Bildgebungsvorrichtung (110), um einen Bildgebungsprozess durchzuführen oder den Bildgebungsprozess zu stoppen.

9. Medizinisches System nach Anspruch 8, weiter umfassend eine Vielzahl von medizinischen Betten (140);
wobei für jedes der Vielzahl von medizinischen Betten (140) die Steuereinheit (130) so konfiguriert ist, dass sie eine Bewegungstrajektorie des medizinischen Bettes (140) basierend auf Umgebungsinformationen innerhalb des medizinischen Raums (170), in dem das medizinische Bett (140) gelegen ist, plant und das medizinische Bett (140) basierend auf der Bewegungstrajektorie bewegt.

10. Medizinisches System nach einem der Ansprüche 1-9, wobei mindestens einer der Vielzahl von medizinischen Räumen (170) einen Hybrid-Operationssaal einschließt.

11. Verfahren zur Steuerung eines medizinischen Systems (100), wobei das medizinische System (100) Folgendes umfasst:
einen Geräteraum (160) und eine Vielzahl von medizinischen Räumen (170);
eine Bildgebungsvorrichtung (110), wobei die Bildgebungsvorrichtung (110) eine bewegliche Bildgebungskomponente (111) einschließt, wobei die Bildgebungskomponente (111) von der Vielzahl von medizinischen Räumen (170) gemeinsam genutzt wird, und wobei die bewegliche Bildgebungskomponente (111) so konfiguriert ist, dass sie sich zwischen dem Geräteraum (160) und den medizinischen Räumen (170) bewegt;
eine Vielzahl von Abschirmtüren (120) für jede der Vielzahl von Abschirmtüren (120), wobei die Abschirmtür (120) zwischen einem der Vielzahl von medizinischen Räumen (170) und dem Geräteraum (160) bereitgestellt ist, wobei
die Bildgebungsvorrichtung (110) einen Sockel (112) einschließt, der zum Tragen der beweglichen Bildgebungskomponente (111) konfiguriert ist,
die bewegliche Bildgebungskomponente (111) mittels eines Roboterarms (113-1), der eine Vielzahl von Freiheitsgraden aufweist, mit dem Sockel (112) verbunden ist,
die bewegliche Bildgebungskomponente (111) in Bezug auf den Sockel (112) bewegbar ist; und
während Bewegung der beweglichen Bildgebungskomponente (111) zwischen dem Geräteraum (160) und der Vielzahl von medizinischen Räumen (170) der Sockel (112) im Geräteraum (160) gelegen ist; und
eine Steuereinheit (130);
wobei das Verfahren Folgendes umfasst:
als Reaktion auf Bestimmen, dass sich die bewegliche Bildgebungskomponente (111) in einen medizinischen Zielraum der Vielzahl von medizinischen Räumen (170) bewegen muss, Steuern durch die Steuereinheit (130) der beweglichen Bildgebungskomponente (111), damit sie sich vom Geräteraum (160) durch eine Zielabschirmungstür der Vielzahl von Abschirmungstüren (120), die dem medizinischen Zielraum entspricht, zum medizinischen Zielraum bewegt;
Steuern durch die Steuereinheit (130) der beweglichen Bildgebungskomponente (111), damit sie einen Scanprozess an einem Objekt im medizinischen Zielraum durchführt; und
als Reaktion auf Bestimmen, dass sich die bewegliche Bildgebungskomponente (111) in den Geräteraum (160) bewegen muss, Steuern durch die Steuereinheit (130) der beweglichen Bildgebungskomponente (111), damit sie sich durch die Zielabschirmungstür vom medizinischen Zielraum in den Geräteraum (160) bewegt.

12. Verfahren nach Anspruch 11, weiter umfassend:
Planen einer Bewegungstrajektorie der beweglichen Bildgebungskomponente (111) und/oder eines medizinischen Zielbetts, wobei das medizinische Zielbett innerhalb des medizinischen Zielraums angeordnet ist; und
Steuern der beweglichen Bildgebungskomponente (111) und/oder des Zielbetts, damit sie sich basierend auf der Bewegungstrajektorie so bewegen, dass eine relative Position zwischen der beweglichen Bildgebungskomponente (111) und dem Zielbett eine Bildgebungs-Scanbedingung erfüllt.

13. Verfahren nach Anspruch 11 oder Anspruch 12, wobei der Sockel (112) innerhalb des Geräteraums (160) fixiert ist.

14. Verfahren nach einem der Ansprüche 11-13, wobei die Vielzahl von medizinischen Räumen (170) um den Geräteraum (160) angeordnet ist.

15. Nichtflüchtiges, computerlesbares Medium, das ausführbare Anweisungen umfasst, wobei die ausführbaren Anweisungen, wenn sie von einem oder mehreren Prozessoren einer Rechenvorrichtung ausgeführt werden, bewirken, dass die Rechenvorrichtung ein Verfahren zum Steuern eines medizinischen Systems (100) durchführt, wobei das medizinische System (100) Folgendes umfasst:
einen Geräteraum (160) und eine Vielzahl von medizinischen Räumen (170);
eine Bildgebungsvorrichtung (110), wobei die Bildgebungsvorrichtung (110) eine bewegliche Bildgebungskomponente (111) einschließt, wobei die Bildgebungskomponente (111) von der Vielzahl von medizinischen Räumen (170) gemeinsam genutzt wird, und wobei die bewegliche Bildgebungskomponente (111) so konfiguriert ist, dass sie sich zwischen dem Geräteraum (160) und den medizinischen Räumen (170) bewegt;
eine Vielzahl von Abschirmtüren (120) für jede der Vielzahl von Abschirmtüren (120), wobei die Abschirmtür (120) zwischen einem der Vielzahl von medizinischen Räumen (170) und dem Geräteraum (160) bereitgestellt ist, wobei
die Bildgebungsvorrichtung (110) einen Sockel (112) einschließt, der zum Tragen der beweglichen Bildgebungskomponente (111) konfiguriert ist,
die bewegliche Bildgebungskomponente (111) mittels eines Roboterarms (113-1), der eine Vielzahl von Freiheitsgraden aufweist, mit dem Sockel (112) verbunden ist,
die bewegliche Bildgebungskomponente (111) in Bezug auf den Sockel (112) bewegbar ist; und
während Bewegung der beweglichen Bildgebungskomponente (111) zwischen dem Geräteraum (160) und der Vielzahl von medizinischen Räumen (170) der Sockel (112) im Geräteraum (160) gelegen ist; und
eine Steuereinheit (130);
wobei das Verfahren Folgendes einschließt:
als Reaktion auf Bestimmen, dass sich die bewegliche Bildgebungskomponente (111) in einen medizinischen Zielraum der Vielzahl von medizinischen Räumen (170) bewegen muss, Steuern durch die Steuereinheit (130) der beweglichen Bildgebungskomponente (111), damit sie sich vom Geräteraum (160) durch eine Zielabschirmungstür der Vielzahl von Abschirmungstüren (120), die dem medizinischen Zielraum entspricht, zum medizinischen Zielraum bewegt;
Steuern durch die Steuereinheit (130) der beweglichen Bildgebungskomponente (111), damit sie einen Scanprozess an einem Objekt im medizinischen Zielraum durchführt; und
als Reaktion auf Bestimmen, dass sich die bewegliche Bildgebungskomponente (111) in den Geräteraum (160) bewegen muss, Steuern durch die Steuereinheit (130) der beweglichen Bildgebungskomponente (111), damit sie sich durch die Zielabschirmungstür vom medizinischen Zielraum in den Geräteraum (160) bewegt.

## Revendications

1. Système médical, comprenant :
une salle d'équipement (160) et une pluralité de salles médicales (170) ;
un dispositif d'imagerie (110), le dispositif d'imagerie (110) incluant un composant d'imagerie mobile (111), le composant d'imagerie mobile (111) étant partagé par la pluralité de salles médicales (170), et le composant d'imagerie mobile (111) étant configuré pour se déplacer entre la salle d'équipement (160) et la pluralité de salles médicales (170) ; et
une pluralité de portes de protection (120), pour chacune de la pluralité de portes de protection (120), la porte de protection (120) étant prévue entre l'une de la pluralité de salles médicales (170) et la salle d'équipement (160), dans lequel
le dispositif d'imagerie (110) inclut une base (112) configurée pour supporter le composant d'imagerie mobile (111),
le composant d'imagerie mobile (111) est relié à la base (112) au moyen d'un bras robotique (113-1) présentant une pluralité de degrés de liberté,
le composant d'imagerie mobile (111) est mobile par rapport à la base (112) ; et
lors du déplacement du composant d'imagerie mobile (111) entre la salle d'équipement (160) et la pluralité de salles médicales (170), la base (112) se trouve dans la salle d'équipement (160).

2. Système médical selon la revendication 1, dans lequel la pluralité de salles médicales (170) sont agencées autour de la salle d'équipement (160).

3. Système médical selon la revendication 1 ou la revendication 2, dans lequel lorsque le composant d'imagerie mobile (111) doit se déplacer vers une salle médicale cible de la pluralité de salles médicales (170),
une porte de protection cible de la pluralité de portes de protection (120) entre la
salle médicale cible et la salle d'équipement (160) est configurée pour être ouverte, et les autres de la pluralité de portes de protection (120) sont configurées pour être fermées.

4. Système médical selon la revendication 3, dans lequel la salle médicale cible et la salle d'équipement (160) forment un espace scellé.

5. Système médical selon la revendication 3 ou la revendication 4, dans lequel les autres de la pluralité de salles médicales (170) sont isolées de la salle médicale cible et de la salle d'équipement (160).

6. Système médical selon l'une quelconque des revendications 1-5, dans lequel le dispositif d'imagerie (110) est un dispositif d'angiographie par soustraction numérique (DSA) monté au sol.

7. Système médical selon l'une quelconque des revendications 1-6, dans lequel la base (112) est fixée à l'intérieur de la salle d'équipement (160).

8. Système médical selon l'une quelconque des revendications 1-7, comprenant en outre un dispositif de commande (130), le dispositif de commande (130) étant configuré pour :
commander l'ouverture ou la fermeture de la pluralité de portes de protection (120), ou commander le déplacement du composant d'imagerie mobile (111) entre la salle d'équipement (160) et les salles médicales (170) ; ou
commander au dispositif d'imagerie (110) de réaliser un processus d'imagerie ou d'arrêter le processus d'imagerie.

9. Système médical selon la revendication 8, comprenant en outre une pluralité de lits médicaux (140) ;
dans lequel pour chacun de la pluralité de lits médicaux (140), le dispositif de commande (130) est configuré pour planifier une trajectoire de déplacement du lit médical (140) en fonction des informations environnementales à l'intérieur de la salle médicale (170) où se trouve le lit médical (140), et pour commander le déplacement du lit médical (140) en fonction de la trajectoire de déplacement.

10. Système médical selon l'une quelconque des revendications 1-9, dans lequel au moins une de la pluralité de salles médicales (170) inclut une salle d'opération hybride.

11. Procédé de commande d'un système médical (100), le système médical (100) comprenant :
une salle d'équipement (160) et une pluralité de salles médicales (170) ;
un dispositif d'imagerie (110), le dispositif d'imagerie (110) incluant un composant d'imagerie mobile (111), le composant d'imagerie mobile (111) étant partagé par la pluralité de salles médicales (170), et le composant d'imagerie mobile (111) étant configuré pour se déplacer entre la salle d'équipement (160) et la pluralité de salles médicales (170) ;
une pluralité de portes de protection (120), pour chacune de la pluralité de portes de protection (120), la porte de protection (120) étant prévue entre l'une de la pluralité de salles médicales (170) et la salle d'équipement (160), dans lequel
le dispositif d'imagerie (110) inclut une base (112) configurée pour supporter le composant d'imagerie mobile (111),
le composant d'imagerie mobile (111) est relié à la base (112) au moyen d'un bras robotique (113-1) présentant une pluralité de degrés de liberté,
le composant d'imagerie mobile (111) est mobile par rapport à la base (112) ; et
lors du déplacement du composant d'imagerie mobile (111) entre la salle d'équipement (160) et la pluralité de salles médicales (170), la base (112) est située dans la salle d'équipement (160) ; et
un dispositif de commande (130) ;
le procédé comprenant :
en réponse à la détermination que le composant d'imagerie mobile (111) doit se déplacer vers une salle médicale cible de la pluralité de salles médicales (170), la commande, par le dispositif de commande (130), du déplacement du composant d'imagerie mobile (111) de la salle d'équipement (160) vers la salle médicale cible à travers une porte de protection cible de la pluralité de portes de protection (120) correspondant à la salle médicale cible ;
la commande, par le dispositif de commande (130), de la réalisation par le composant d'imagerie mobile (111) d'un processus de numérisation sur un objet dans la salle médicale cible ; et
en réponse à la détermination que le composant d'imagerie mobile (111) doit se déplacer vers la salle d'équipement (160), la commande, par le dispositif de commande (130), du déplacement du composant d'imagerie mobile (111) de la salle médicale cible vers la salle d'équipement (160) à travers la porte de protection cible.

12. Procédé selon la revendication 11, comprenant en outre :
la planification d'une trajectoire de déplacement du composant d'imagerie mobile (111) et/ou d'un lit médical cible, le lit médical cible étant disposé à l'intérieur de la chambre médicale cible ; et
la commande du déplacement du composant d'imagerie mobile (111) et/ou du lit médical cible en fonction de la trajectoire de déplacement afin qu'une position relative entre le composant d'imagerie mobile (111) et le lit médical cible satisfasse une condition de numérisation d'imagerie.

13. Procédé selon la revendication 11 ou la revendication 12, dans lequel la base (112) est fixée à l'intérieur de la salle d'équipement (160).

14. Procédé selon l'une quelconque des revendications 11-13, dans lequel la pluralité de salles médicales (170) sont agencées autour de la salle d'équipement (160).

15. Support non transitoire lisible par ordinateur, comprenant des instructions exécutables, dans lequel, lorsqu'elles sont exécutées par un ou plusieurs processeurs d'un dispositif informatique, les instructions exécutables amènent le dispositif informatique à réaliser un procédé de commande d'un système médical (100), le système médical (100) comprenant :
une salle d'équipement (160) et une pluralité de salles médicales (170) ;
un dispositif d'imagerie (110), le dispositif d'imagerie (110) incluant un composant d'imagerie mobile (111), le composant d'imagerie mobile (111) étant partagé par la pluralité de salles médicales (170), et le composant d'imagerie mobile (111) étant configuré pour se déplacer entre la salle d'équipement (160) et la pluralité de salles médicales (170) ;
une pluralité de portes de protection (120), pour chacune de la pluralité de portes de protection (120), la porte de protection (120) étant prévue entre l'une de la pluralité de salles médicales (170) et la salle d'équipement (160), dans lequel
le dispositif d'imagerie (110) inclut une base (112) configurée pour supporter le composant d'imagerie mobile (111),
le composant d'imagerie mobile (111) est relié à la base (112) au moyen d'un bras robotique (113-1) présentant une pluralité de degrés de liberté,
le composant d'imagerie mobile (111) est mobile par rapport à la base (112) ; et
lors du déplacement du composant d'imagerie mobile (111) entre la salle d'équipement (160) et la pluralité de salles médicales (170), la base (112) est située dans la salle d'équipement (160) ; et
un dispositif de commande (130) ;
le procédé incluant :
en réponse à la détermination que le composant d'imagerie mobile (111) doit se déplacer vers une salle médicale cible de la pluralité de salles médicales (170), la commande, par le dispositif de commande (130), du déplacement du composant d'imagerie mobile (111) de la salle d'équipement (160) vers la salle médicale cible à travers une porte de protection cible de la pluralité de portes de protection (120) correspondant à la salle médicale cible ;
la commande, par le dispositif de commande (130), de la réalisation par le composant d'imagerie mobile (111) d'un processus de numérisation sur un objet dans la salle médicale cible ; et
en réponse à la détermination que le composant d'imagerie mobile (111) doit se déplacer vers la salle d'équipement (160), la commande, par le dispositif de commande (130), du déplacement du composant d'imagerie mobile (111) de la salle médicale cible vers la salle d'équipement (160) à travers la porte de protection cible.
